(19) Europäisches Patentamt
European Patent Office
Office européen des brevets

(11) Veröffentlichungsnummer: **0 429 558 B1**

(12) **EUROPÄISCHE PATENTSCHRIFT**

(45) Veröffentlichungstag der Patentschrift: **30.03.94**

(51) Int. Cl.$^5$: **C07C 25/18**, C09K 19/14, C07C 43/225, C07C 255/50, C09K 19/30

(21) Anmeldenummer: **90902769.0**

(22) Anmeldetag: **22.01.90**

(86) Internationale Anmeldenummer:
**PCT/EP90/00120**

(87) Internationale Veröffentlichungsnummer:
**WO 90/08757 (09.08.90 90/19)**

(54) **BIPHENYLYLETHANE UND FLÜSSIGKRISTALLINE PHASE.**

(30) Priorität: **27.01.89 DE 3902328**
**27.01.89 DE 3902330**
**25.04.89 DE 3913554**

(43) Veröffentlichungstag der Anmeldung:
**05.06.91 Patentblatt 91/23**

(45) Bekanntmachung des Hinweises auf die Patenterteilung:
**30.03.94 Patentblatt 94/13**

(84) Benannte Vertragsstaaten:
**AT BE CH DE ES FR GB IT LI LU NL SE**

(56) Entgegenhaltungen:
**EP-A- 0 149 208**
**EP-A- 0 227 004**
**FR-A- 2 308 676**

(73) Patentinhaber: **MERCK PATENT GmbH**
**Postfach,**
**Frankfurter Strasse 250**
**D-64271 Darmstadt(DE)**

(72) Erfinder: **POETSCH, Eike, Dr.**
**Am Buchwald 4**
**D-6109 Mühltal 6(DE)**
Erfinder: **MEYER, Volker**
**Semder Pfad 23**
**D-6112 Gross-Zimmern 2(DE)**
Erfinder: **STAHL, Klaus-Peter, Dr.**
**Kurt-Schumacher-Str. 50**
**D-6100 Darmstadt 13(DE)**
Erfinder: **REIFFENRATH, Volker**
**Jahnstr. 15**
**D-6101 Rossdorf(DE)**
Erfinder: **FINKENZELLER, Ulrich, Dr.**
**Waldpfad 74**
**D-6831 Plankstadt(DE)**

**EP 0 429 558 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**JOURNAL OF CHROMATOGRAPHY, Band 455, 1988, Elsevier Science Publishers B.V., Amsterdam, NL; J. MAZUR et al: "Isothiocyanate as a liquid crystalline stationary phase in capillary gas chromatography for the separation of polyaromatic hydrocarbons", Seite 323-326**

**J. CHEMICAL SOCIETY, Perkin Transactions II, Band 7, 1976, The Chemical Society; D. COATES et al: "Properties of the liquid crystals formed by some 4'-substituted 4-(beta-p-substituted arylethyl)biphenyls", Seite 863-868**

**MOL. CRYST, LIO. CRYST., Band 124, (1-4), 1985, Gordon and Breach, Science Publishers, Inc. and OPA Ltd., USA; J. E. FEARON et al: "The effect of lateral fluoro-substitution on the liquid crystalline properties of some 4-n-alkyl-, 4-n-alkoxy- and related 4-substituted-4'-cyanobiphenyls", Seite 89-103**

Erfinder: **BARTMANN, Ekkehard, DR.**
**Dieburger Weg 12a**
**D-6106 Erzhausen(DE)**
Erfinder: **HITTICH, Reinhard, Dr.**
**Am Kirchberg 11**
**D-6101 Modautal 1(DE)**
Erfinder: **COATES, David, Dr. 87 Sopwith Crescent**
**Merley, Wimborne**
**Dorset BH21 3SW(GB)**
Erfinder: **GREENFIELD, Simon, Dr. 2 Blackbird Close Creekmoor**
**Poole**
**Dorset BH17 7YA(GB)**
Erfinder: **SMITH, Graham, Dr.**
**10 Henbury Close**
**Canford Heath**
**Poole**
**Dorset BH17 8AX(GB)**
Erfinder: **KURMEIER, Hans-Adolf, Dr.**
**Hinter der Schule 3a**
**D-6104 Seeheim-Jugenheim(DE)**
Erfinder: **DORSCH, Dieter, Dr.**
**Reuterallee 24**
**D-6100 Darmstadt(DE)**

**Beschreibung**

Die Erfindung betrifft Biphenylylethane der Formel I

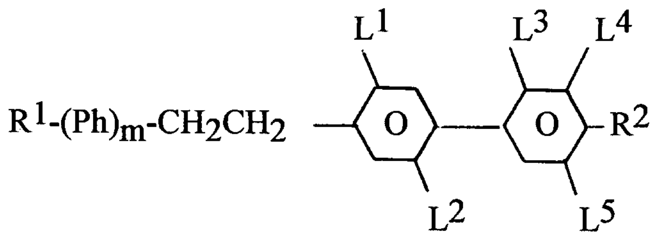

I

worin einer der Reste $R^1$ und $R^2$ X und der andere Rest $R^1$ bzw. $R^2$ R-Y- bedeutet, wobei

| | |
|---|---|
| X | H, F, Cl, $-CF_3$, $-OCHF_2$, $-OCF_3$, -CN, -NCS oder $-NO_2$, |
| R | Alkyl, Fluoralkyl, Alkenyl oder Oxaalkyl und |
| Y | O, S, -CO-O-, -O-CO-, -O-CO-O-, eine Einfachbindung, oder - im Falle m = 1 - auch trans-1,4-Cyclohexylen oder $C_2H_4$- bedeutet, |
| m | 1 oder 2, |
| Ph | jeweils gleiche oder verschiedene Reste ausgewählt aus der Gruppe bestehend aus 1,4-Phenylen, 2-Fluor-1,4-phenylen, 3-Fluor-1,4-phenylen und 3,5-Difluor-1,4-phenylen, |
| $L^3$ und $L^4$ | jeweils H, oder einer dieser Reste auch F, |
| $L^1$,$L^2$ und $L^5$ | jeweils H oder F bedeutet, mit den Maßgaben, daß |

(a) im Falle

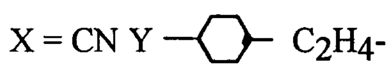

bedeutet und/oder m = 2 und/oder $L^1$ Fluor bedeutet und/oder Ph-CN, 2-Fluor-4-cyanphenyl oder 3,5-Difluor-4-cyanphenyl bedeutet und

(b) im Falle

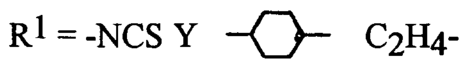

bedeutet und/oder m = 2 und/oder einer der Reste $L^1$, $L^2$ und $L^3$ Fluor bedeutet,

sowie Biphenylylethane der Formel I36

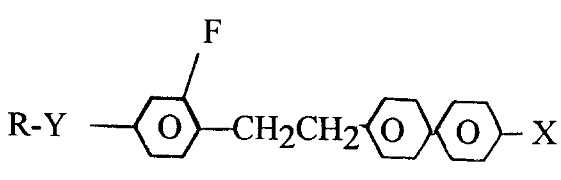

I36

worin R und Y die oben angegebene Bedeutung haben und X -CN oder -NCS bedeutet.

Die Verbindungen der Formel I können als Komponenten flüssigkristalliner Phasen verwendet werden, insbesondere für Displays, die auf dem Prinzip der verdrillten Zelle einschließlich höher verdrillter Zellen, dem Guest-Host-Effekt, dem Effekt der Deformation aufgerichteter Phasen oder dem Effekt der dynamischen Streuung beruhen.

Ähnliche Verbindungen sind z.B. aus den DOS 30 40 632 und 34 01 320 bekannt. Die dort beschriebenen Verbindungen sind jedoch keine Biphenylylethane, sondern Cyclohexylphenylethane.

Verbindungen der Formel,

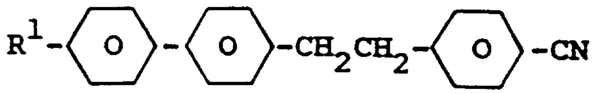

worin $R^1$ n-Alkyl oder n-Alkoxy bedeutet, sind aus der DOS 26 17 593 bekannt. Verbindungen derselben Formel, worin $R^1$ verzweigtes Alkyl bedeutet, sind aus DOS 27 36 772 als thermochrome Materialien bzw. aus JP 62-146984 als Materialien für ferroelektrische $S_c$-Mischungen bekannt.

Verbindungen der Formel,

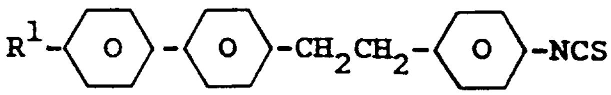

worin $R^1$ n-Pentyloxy, 2-Methylbutyl, n-Butyl, n-Pentyl, n-Hexyl und n-Heptyl bedeuten, sind aus der EP-OS 0 227 004 bekannt.

Weitere ähnliche Verbindungen sind bekannt aus I. Mazur et al., Journal of Chromatography, Bd. 455, 1988, S. 323-226; D. Coates et al., I. Chem. Soc., Perkin Transaetions II, Bd. 7, 1976, S. 863-868 und I. E. Feron et al., Mol. Cryst. Liq. Cryst., Bd. 1,124(1-4), 1985, S. 89-103.

Der Erfindung lag die Aufgabe zugrunde, neue stabile flüssigkristalline oder mesogene Verbindungen mit hoher Doppelbrechung aufzufinden, die als Komponenten flüssigkristalliner Phasen geeignet sind. Diese Aufgabe wurde durch die Bereitstellung der Verbindungen der Formel I gelöst.

Es wurde gefunden, daß die Verbindungen der Formel I als Komponenten flüssigkristalliner Phasen vorzüglich geeignet sind. Insbesondere sind mit ihrer Hilfe stabile flüssigkristalline Phasen mit relativ großer optischer Anisotropie, positiver dielektrischer Anisotropie, geringer Viskosität und hoher Nematogenität in Verbindung mit günstigem Tieftemperaturvehalten herstellbar. Die Substanzen der Formel I sind beispielsweise besonders bevorzugt für die Verwendung in Mischungen für Supertwist-Effekte oder für Anzeigen mit aktiver Matrix geeignet.

Überraschend zeigte sich, daß der Zusatz von Verbindungen der Formel I flüssigkristalline Phasen liefert, die alle oben genannten Kriterien hervorragend erfüllen.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu optimieren. Die Verbindungen der Formel I eignen sich ferner als Zwischenprodukte zur Herstellung anderer Substanzen, die sich als Bestandteile flüssigkristalliner Phasen verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie die Verwendung dieser Verbindungen als Komponenten flüssigkristalliner Phasen. Weiterhin sind Gegenstand der Erfindung flüssigkristalline Phasen mit einem Gehalt an mindestens einer Verbindung der Formel I, sowie Flüssigkristall-Anzeigeelemente, die derartige Phasen enthalten.

Vor- und nachstehend haben $R^1$, $R^2$, m, Y, Ph, $L^1$, $L^2$, $L^3$, $L^4$, $L^5$ und X die angegebene Bedeutung, sofern nicht ausdrücklich etwas anderes vermerkt ist.

Besonders bevorzugt sind Biphenylethane der Formel Ia,

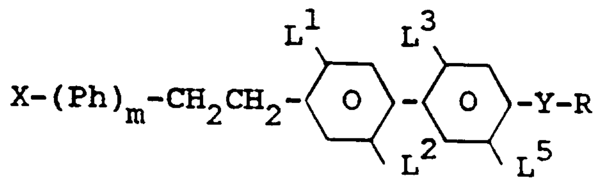

Ia

worin

| | |
|---|---|
| R | Alkyl, Fluoralkyl, Alkenyl oder Oxaalkyl, |
| m | 1 oder 2, |
| Y | O, S, CO-O, O-CO, O-CO-O, eine Einfachbindung, oder - im Falle m = 1 - auch trans-1,4-Cyclohexylen, |
| Ph | jeweils gleiche oder verschiedene Reste ausgewählt aus der Gruppe bestehend aus 1,4-Phenylen, 2-Fluor-1,4-phenylen, 3-Fluor-1,4-phenylen und 3,5-Difluor-1,4-phenylen, |
| $L^1$, $L^2$, $L^3$ und $L^5$ | jeweils H oder F, |
| X | F, Cl, $-CF_3$, -CN, $-OCF_3$, $-OCHF_2$, -NCS oder $-NO_2$ bedeutet, mit der Maßgabe, daß |

a) im Falle X = -CN Y trans-1,4-Cyclohexylen oder m = 2 und/oder Ph-X 2-Fluor-4-cyanphenyl, 3-Fluor-4-cyanphenyl oder 3,5-Difluor-4-cyanphenyl und/oder einer oder zwei der Reste $L^1$, $L^2$, $L^3$ und $L^5$ F bedeutet, und

b) im Falle X = NCS m = 2 und/oder Ph-X 2-Fluor-4-isothiocyanatophenyl, 3-Fluor-4-isothiocyanatophenyl oder 3,5-Difluor-4-isothiocyanatophenyl und/oder einer oder zwei der Reste $L^1$, $L^2$, $L^3$ und $L^5$ F und/oder -Y-R geradkettiges Alkyl mit bis zu 3 C-Atomen bedeutet,

sowie die Biphenylylethane der Formel Ib,

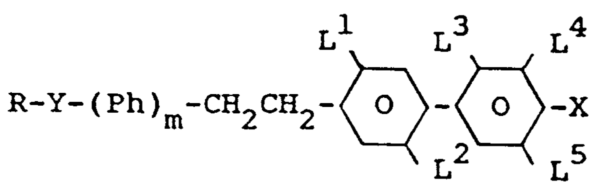

Ib

worin

| | |
|---|---|
| R | Alkyl, Fluoralkyl, Alkenyl oder Oxaalkyl, |
| m | 1 oder 2, |
| Y | O, S, CO-O, O-CO, O-CO-O, eine Einfachbindung, oder - im Falle m = 1 - auch trans-1,4-Cyclohexylen, |
| Ph | jeweils gleiche oder verschiedene Reste ausgewählt aus der Gruppe bestehend aus 1,4-Phenylen, 2-Fluor-1,4-phenylen, 3-Fluor-1,4-phenylen und 3,5-Difluor-1,4-phenylen, |
| $L^1$, $L^3$ und $L^4$ | jeweils H, oder einer dieser Reste auch F, |
| $L^2$ und $L^5$ | jeweils H oder F, und |
| X | F, Cl, $-CF_3$, -CN, $-OCF_3$, -NCS oder $-NO_2$ bedeutet, mit der Maßgabe, daß |

im Falle X = F, Cl, -CN oder $-CF_3$ m = 2 und/oder $L^4$ und/oder $L^5$ Fluor (falls X = Halogen oder $-CF_3$) bzw. $L^4$ und $L^5$ oder $L^1$ (falls X = CN) Fluor bedeutet.

Die Verbindungen der Formel Ia umfassen dementsprechend Verbindungen der folgenden bevorzugten Teilformeln:

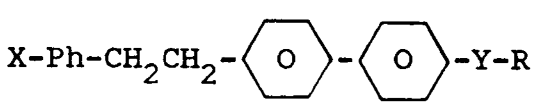

Ia1

worin X, Ph und R die angegebene Bedeutung haben und Y 0 oder eine Einfachbindung bedeutet.

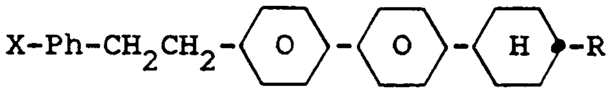

Ia2

worin X, Ph und R die angegebene Bedeutung haben.

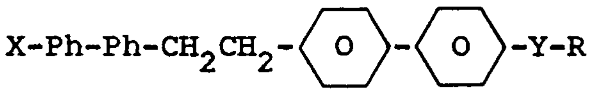

Ia3

Die Verbindungen der Formel Ib umfassen dementsprechend Verbindungen der folgenden bevorzugten Teilformeln:

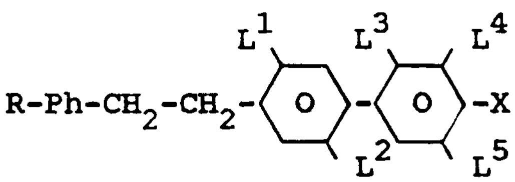

Ib1

worin R, Ph, $L^1$, $L^2$, $L^3$, $L^4$, $L^5$ und X die angegebene Bedeutung haben.

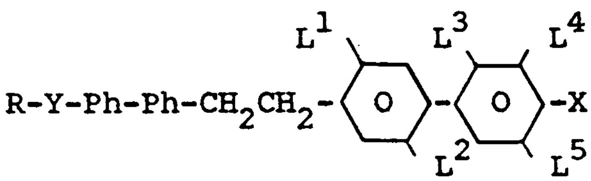

Ib2

worin R, Ph, $L^1$, $L^2$, $L^3$, $L^4$, $L^5$ und X die angegebene Bedeutung haben.

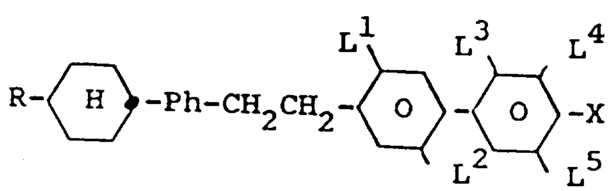

Ib3

worin R, Ph, $L^1$, $L^2$, $L^3$, $L^4$, $L^5$ und X die angegebene Bedeutung haben.

In Formel Ib1 bedeutet X vorzugsweise $-OCF_3$, -NCS oder $NO_2$. Besonders bevorzugt sind Verbindungen der Formel Ib1, worin X F, Cl oder $CF_3$ und $L^4$ und/oder $L^5$ F bedeutet.

Bevorzugt sind auch Verbindungen der Formel Ib1, worin X CN und $L^4$ und $L^5$ Fluor oder X CN und einer der Reste $L^1$, $L^2$ und $L^3$ Fluor bedeutet.

In der Formel Ib2 und Ib3 bedeutet X vorzugsweise -CN, F, Cl, $-CF_3$ oder $-OCF_3$.

In Formel I sowie in den Teilformeln bedeutet X vorzugsweise F, Cl, $-CF_3$, $-OCF_3$, $OCHF_2$, -NCS oder $-NO_2$. Ph-X ist vorzugsweise 3-Fluor-4-X-phenyl oder 3,5-Difluor-4-X-phenyl. Besonders bevorzugte Bedeutungen sind 2-Fluor-4-cyanphenyl, 3-Fluor-4-cyanphenyl, 3,5-Difluor-4-cyanphenyl, 2-Flour-4-isothiocyanatophenyl, 3-Fluor-4-isothiocyanatophenyl und 3,5-Difluor-4-isothiocyanatophenyl.

Besonders bevorzugt sind ferner Verbindungen der Teilformel Ia, worin X -CN oder -NCS und einer der Reste $L^1$, $L^2$, $L^3$ und $L^5$ F bedeutet.

Ebenfalls bevorzugt sind Biphenylylethane der Formel Ia2, worin X -CN oder -NCS bedeutet, sowie Biphenylylethane der Formel Ia3, worin X -CN Oder -NCS bedeutet.

Besonders bevorzugt sind auch die Verbindungen der Formel Ia4,

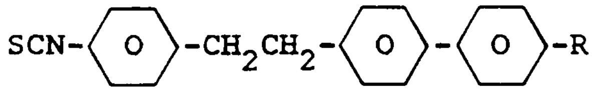

Ia4

worin R Methyl, Ethyl oder n-Propyl bedeutet.

Die Reste R haben vorzugsweise bis zu 10 C-Atome, insbesondere 2 bis 7 C-Atome.

Falls die Gruppen R Alkylreste bedeuten, in denen auch eine ("Oxaalkyl") $CH_2$-Gruppe durch O-Atome ersetzt sein kann, so können sie geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, Ethoxy, Propoxy, Butoxy, Pentoxy, Hexoxy, Heptoxy, 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy, Tetradecoxy, Pentadecoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Besonders bevorzugt sind auch Alkylreste in denen eine $CH_2$-Gruppe durch eine -CH = CH-Gruppe oder durch -CHF- ersetzt ist.

m ist vorzugsweise 1, X ist vorzugsweise 0, eine Einfachbindung oder trans-1,4-Cyclohexylen; insbesondere bevorzugt eine Einfachbindung. Y ist vorzugsweise trans-1,4-Cyclohexylen, -O- oder eine Einfachbindung.

Verbindungen der Formel I mit verzweigten Flügelgruppen R können gelegentlich wegen einer besseren Löslichkeit in den üblichen flüssigkristallinen Basismaterialien von Bedeutung sein, insbesondere aber als chirale Dotierstoffe, wenn sie optisch aktiv sind.

Verzweigte Gruppen dieser Art enthalten in der Regel nicht mehr als eine Kettenverzweigung. Bevorzugte verzweigte Rest sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy (= 2-Octyloxy), 2-Oxa-3-methylbutyl, 3-Oxa-4-methylpentyl, 4-Methylhexyl, 2-Nonyl, 2-Decyl, 2-Dodecyl, 6-Methyloctoxy, 6-Methyloctanoyloxy, 5-Methylheptyloxy carbonyl, 2-Methylbutyryloxy, 3-Methylvaleryloxy, 4-Methylhexanoyloxy, 2-Methyl-3-oxapentyl, 2-Methyl-3-oxahexyl.

Bei Verbindungen mit verzweigten Flügelgruppen umfaßt Formel I sowohl die optischen Antipoden als auch Racemate sowie deren Gemische.

Unter den Verbindungen der Formel I und deren Unterformeln sind diejenigen bevorzugt, in denen mindestens einer der darin enthaltenen Reste eine der angegebenen bevorzugten Bedeutungen hat.

Besonders bevorzugte kleinere Gruppen von erfindungsgemäßen Verbindungen sind diejenigen der folgenden Teilformeln:

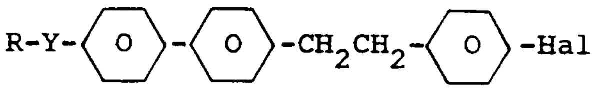
I1

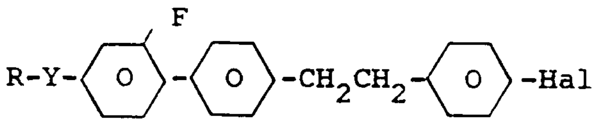
I2

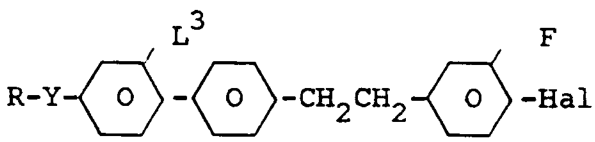
I3

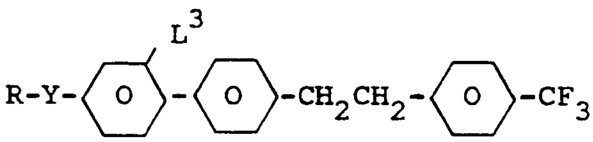
I4

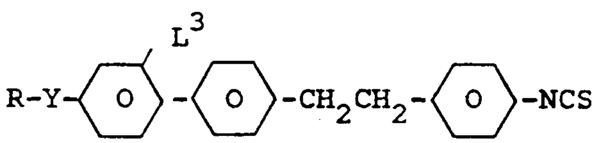
I5

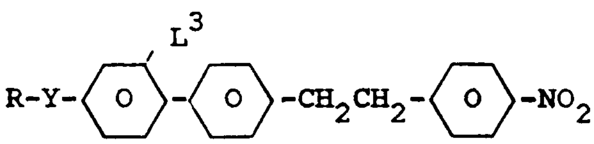
I6

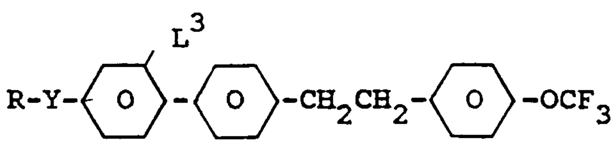
I7

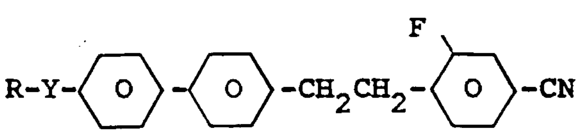
I8

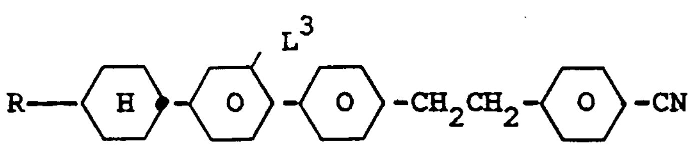
I9

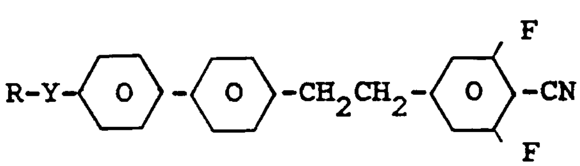
I10a

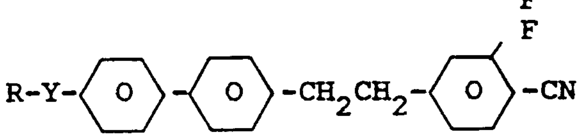
I10b

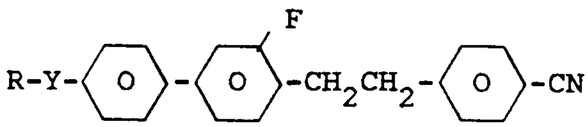

I11a

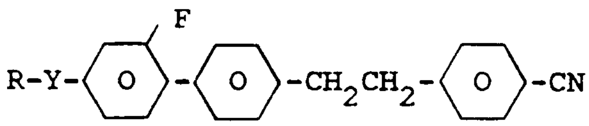

I11b

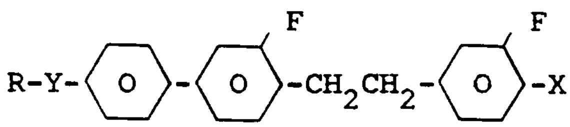

I12

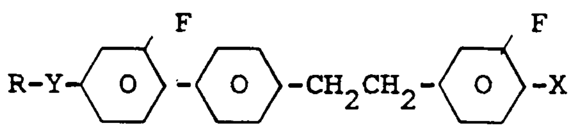

I13

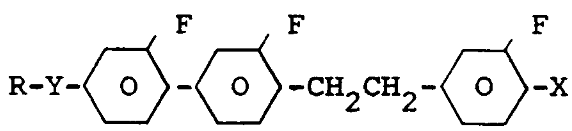

I14

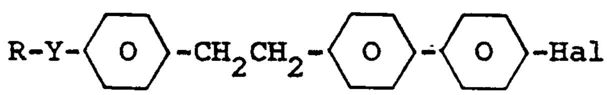

I15

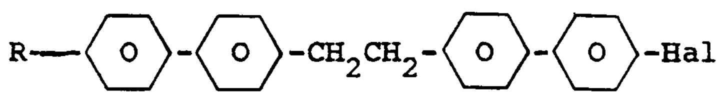

I16

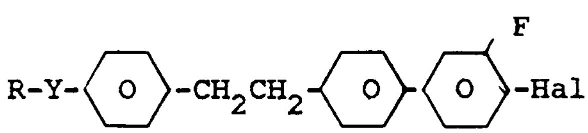

I17

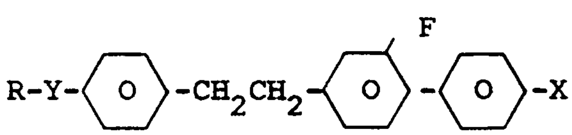

I18

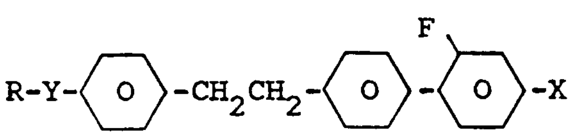

I19

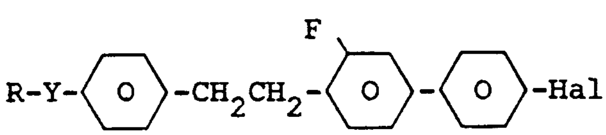

I20

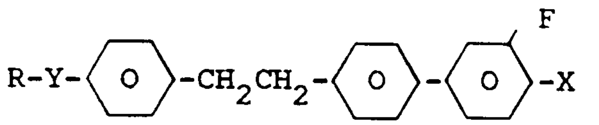
I21

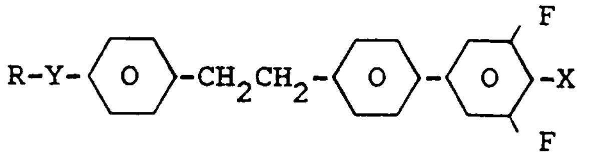
I22

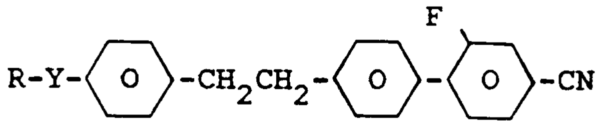
I23

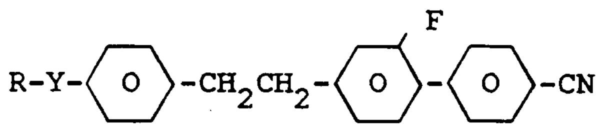
I24

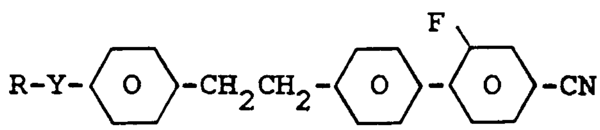
I25

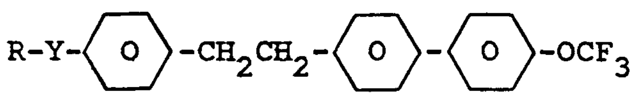
I26

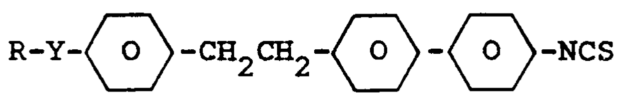
I27

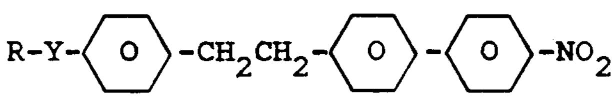
I28

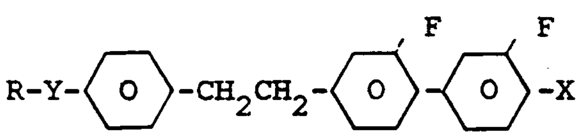
I29

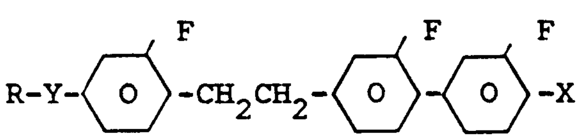
I30

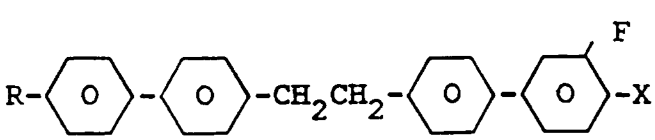
I31

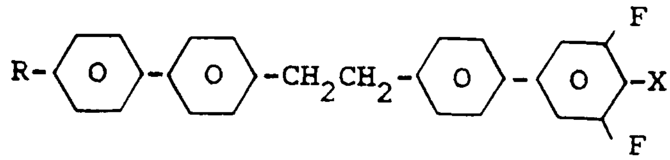

I32

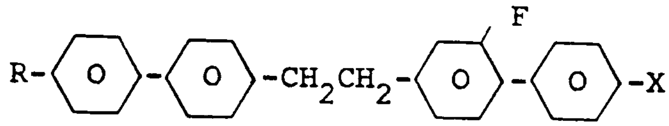

I33

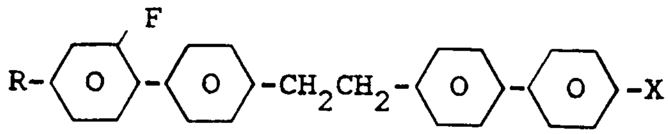

I34

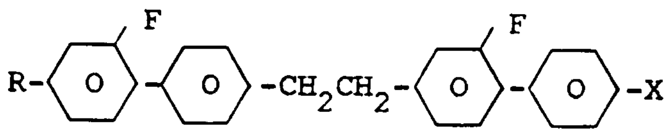

I35

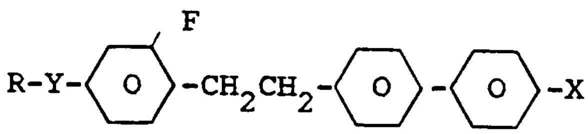

I36

Unter den Verbindungen der Formel I und deren Unterformeln sind diejenigen bevorzugt, in denen mindestens einer der darin enthaltenen Reste ein der angegebenen bevorzugten Bedeutungen hat.

Besonders bevorzugt sind die Verbindungen der Formel

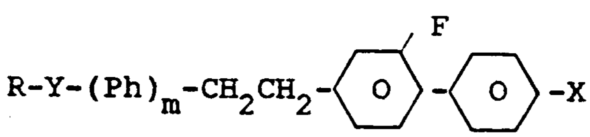

V

worin X vorzugsweise F, Cl, $CF_3$, CN, $OCF_3$ oder $OCHF_2$ bedeutet. Diese Verbindungen sind gemäß folgender Syntheseschemata erhältlich:

X = CN:

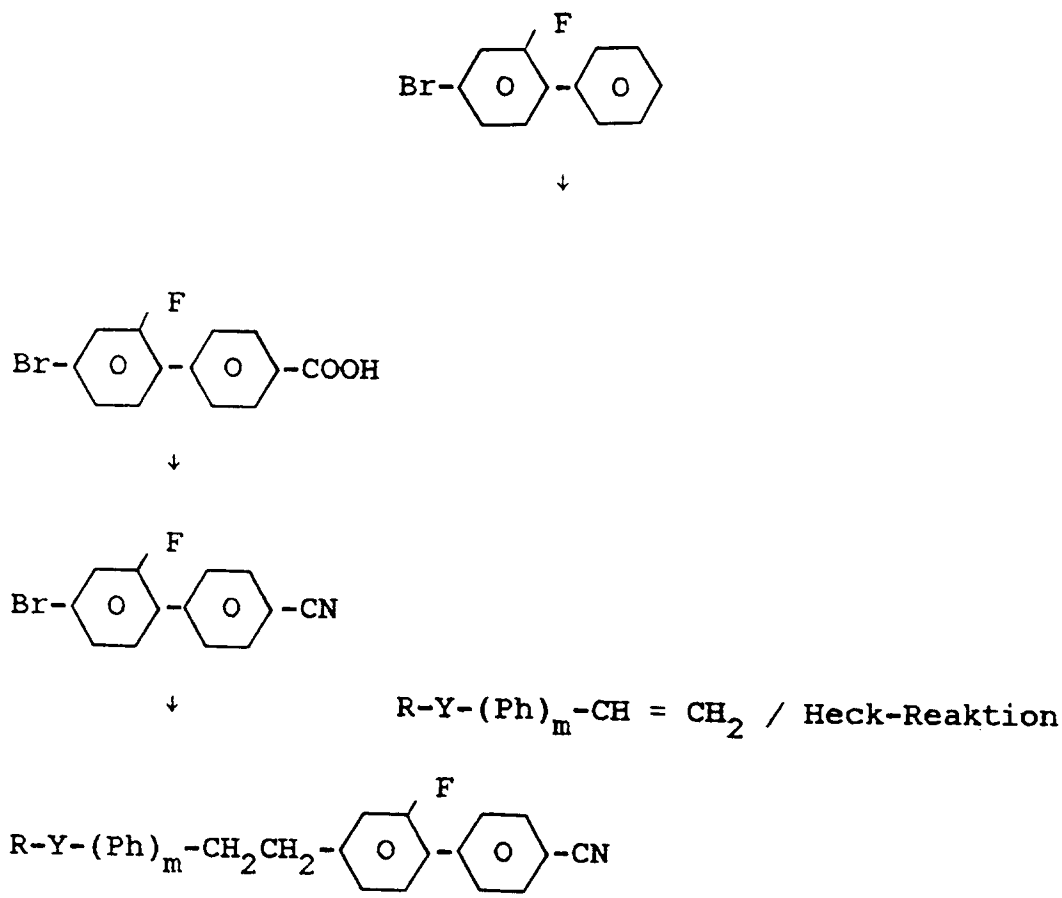


X = $OCHF_2$:

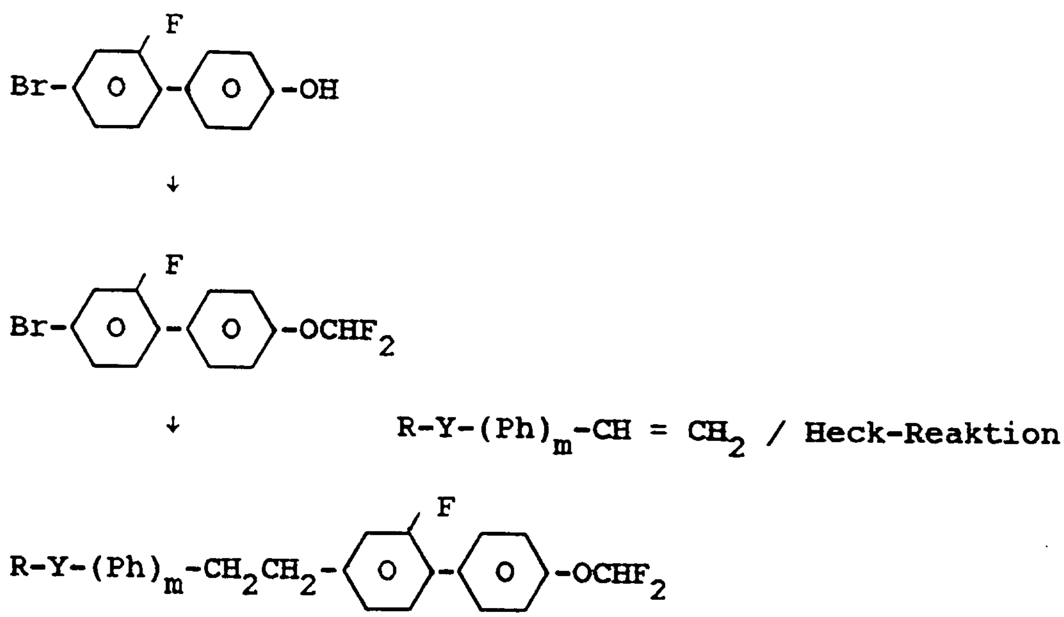

X = F, Cl, $OCF_3$, $CF_3$:

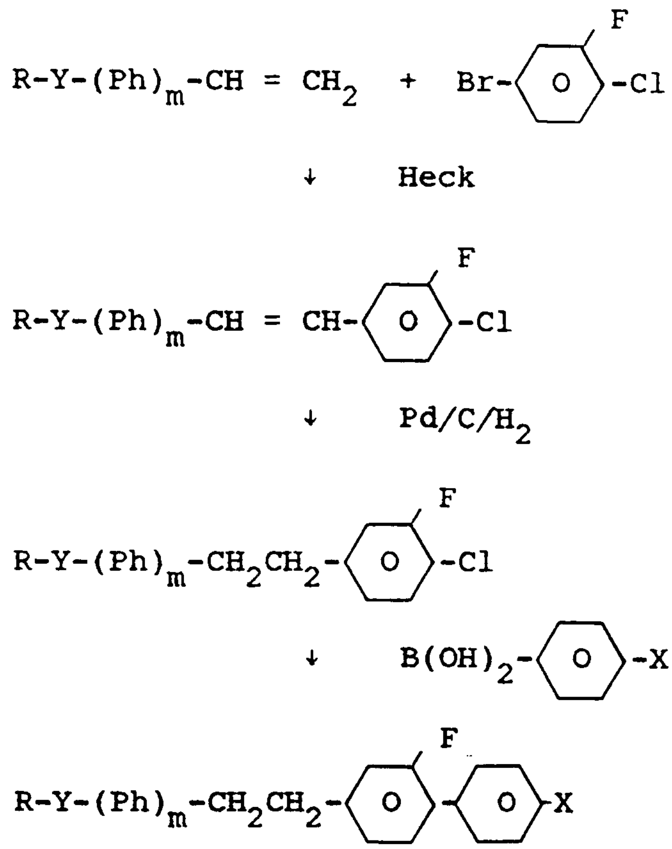


Bezüglich der lateralen Substitution durch Fluor sind die folgenden Substitutionsmuster besonders bevorzugt:

| $R^1$-$(Ph)_m$- | $L^1$ | $L^2$ | $L^3$ | $L^4$ | $L^5$ |
|---|---|---|---|---|---|
| R-Y-$(Ph)_m$- | H | F | H | H | H |
| R-Y-$(Ph)_m$- | H | H | H | F | H |
| R-Y-$(Ph)_m$- | H | H | H | F | F |
| R-Y-$(Ph)_m$- | H | F | H | F | H |
| R-Y-$(Ph)_m$- | H | F | H | F | F |

| | | | | | |
|---|---|---|---|---|---|
| 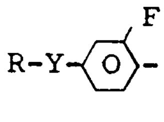 | H | H | H | H | H |
| 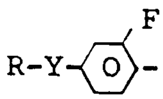 | H | F | H | H | H |
| $X-(Ph)_m$ | H | H | F | H | H |
| $X-(Ph)_m$ | F | H | H | H | H |
| $X-(Ph)_m$ | F | H | F | H | H |
| 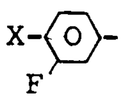 | H | H | H | H | H |
| 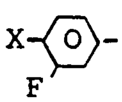 | H | H | F | H | H |

Besonders bevorzugt sind Biphenylylethane der Formel I

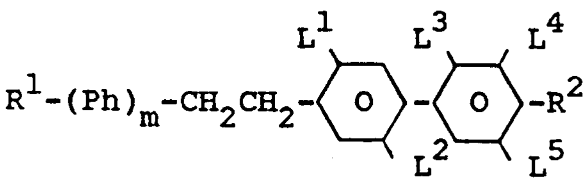

I

worin einer der Reste $R^1$ und $R^2$ X und der andere Rest $R^1$ bzw. $R^2$ R-Y- bedeutet, wobei

X H, Halogen, $-CF_3$, $-OCHF_2$, $-OCF_3$, -CN, -NCS oder $-NO_2$,

R Alkyl, Fluoralkyl, Alkenyl oder Oxaalkyl und

Y O, S, -CO-O-, -O-CO-, -O-CO-O-, eine Einfachbindung, oder - im Falle m = 1 - auch trans-1,4-Cyclohexylen oder

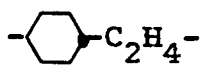

bedeutet,

m 1 oder 2,

Ph jeweils gleiche oder verschiedene Reste ausgewählt aus der Gruppe bestehend aus 1,4-Phenylen, 2-Fluor-1,4-phenylen, 3-Fluor-1,4-phenylen und 3,5-Difluor-1,4-phenylen,

$L^3$ und $L^4$ jeweils H, oder einer dieser Reste auch F,

$L^1$, $L^2$ und $L^5$ jeweils H oder F bedeuten, mit den Maßgaben, daß

(1) bei $R^2$ = X

im Falle X = Halogen, -CN oder $CF_3$, Y trans-1,4-Cyclohexylen oder

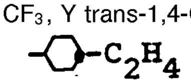

bedeutet oder m = 2 und/oder $L^4$ und/oder $L^5$ Fluor (falls X = Halogen oder $-CF_3$)

bzw. $L^4$ und $L^5$ oder einer der Reste $L^1$, $L^2$ und $L^3$ (falls X = CN) Fluor bedeutet, oder

(2) bei $R^1$ = X

a) im Falle X = -CN Y trans-1,4-Cyclohexylen oder

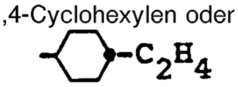

bedeutet oder m = 2 und/oder Ph-X 2-Fluor-4-cyanphenyl, 3-Fluor-4-cyanphenyl oder 3,5-Difluor-4-cyanphenyl und/oder einer oder zwei der Reste $L^1$, $L^2$, $L^3$ und $L^4$ F bedeutet, und

b) im Falle X = NCS, Y = trans-1,4-Cyclohexylen oder

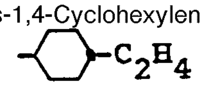

bedeutet oder m = 2 und/oder Ph-X 2-Fluor-4-isothiocyanatophenyl, 3-Fluor-4-isothiocyanatophenyl oder 3,5-Difluor-4-isothiocyanatophenyl und/oder einer oder zwei der Reste $L^1$, $L^2$, $L^3$ und $L^4$ F und/oder -Y-R geradkettiges Alkyl mit bis zu 3 C-Atomen bedeutet.

Besonders bevorzugt sind Verbindungen der Teilformel Ib, worin Y trans-1,4-Cyclohexylen oder

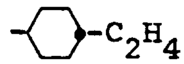

bedeutet oder m 2 ist.

Bevorzugt sind diejenigen Verbindungen der Formel Ib, worin $L^4$ und/oder $L^5$ Fluor oder einer der Reste $L^1$, $L^2$ und $L^3$ Fluor bedeutet. Im ersten Fall ($L^4$ und/oder $L^5$ = F) ist X vorzugsweise Halogen oder $CF_3$. Im zweiten Fall ist X vorzugsweise CN.

Diejenigen Verbindungen der Teilformel Ia sind bevorzugt, worin Y trans-1,4-Cyclohexylen oder

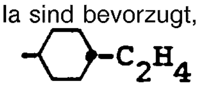

bedeutet oder m 2 ist. Ph-X ist vorzugsweise 2-Fluor-4-cyanphenyl oder 3,5-Difluor-4-cyanphenyl falls X = CN. Bei Teilformel Ia ist vorzugsweise einer oder zwei der Reste $L^1$, $L^2$, $L^3$ und $L^4$ F.

Die Verbindungen der Formel I werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung der Formel II

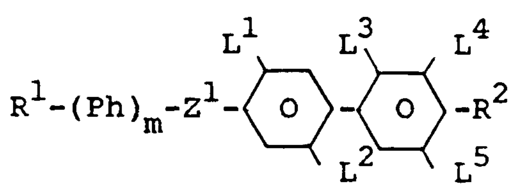

II

worin $Z^1$ -CH=CH- oder -C≡C- bedeutet,

unter dem Fachmann bekannten Bedingungen katalytisch hydriert. In Formel II haben $L^1$, $L^2$, $L^3$, $L^4$, $L^5$, m, $R^1$ und $R^2$ die oben angegebenen Bedingungen. R ist Alkyl, Fluoralkyl oder Oxaalkyl.

Die Verbindungen der Formel II sind erhältlich in an sich bekannter Weise nach Wittig durch Umsetzung entsprechender Benzadehyde mit entsprechenden Phosphoryliden oder durch Heckkopplung aus entsprechenden Styrol-Verbindungen mit Halogenaromaten (Brom oder Jod), welche entweder bekannt sind oder in völliger Analogie zu bekannten Verbindungen hergestellt werden können.

Tolane der Formel II können auch hergestellt werden über die Kopplung von Alkinyl-Zink-Verbindungen mit Arylhalogeniden analog dem von A.O. King, E. Negishi, F.J. Villani und A. Silveira in J. Org. Chem. 43 (1978) 358 beschriebenen Verfahren.

Tolane der Formel II können auch über die Fritsch-Buttenberg-Wiechell-Umlagerung (Ann. 279, 319, 327, 332, 1984) hergestellt werden, bei der 1,1-Diaryl-2-halogenethylene umgelagert werden zu Diarylacetylenen in Gegenwart starker Basen.

Tolane der Formel II können weiterhin hergestellt werden aus 4-substituierten Phenyl- oder Cyclohexylacetylenen und Arylhalogeniden in Gegenwart eines Palladiumkatalysators, z.B. Bis(triphenylphosphin)-palladium(II)-chlorid und Kupfer(I)-jodid (beschriebenen in Synthesis (1980) 627 oder Tetrahedron Letters 27 (1986) 1171).

Die als Ausgangsmaterialien dienenden Verbindungen, z.B. die Materialien der Formeln III und IV

$R^1$-$(Ph)_m$-$Q^1$

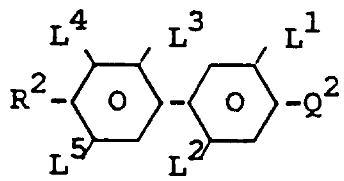

worin $Q^1$ und $Q^2$ beispielsweise $-CH_2COCl$, $-CH=CH_2$, -CHO oder $-CH_2Br$ bedeuten, sind bekannt oder können in völliger Analogie zu bekannten Verbindungen nach Standardmethoden hergestellt werden.

Erfindungsgemäße Verbindungen mit Alkenylgruppen lassen sich in Analogie zu ähnlichen, bekannten Verbindungen durch Wittig-Reaktion darstellen, wobei die Ausgangsmaterialien (entsprechend Formel I, jedoch einer der Reste $R^1$ und $R^2$ ist $-(CH_2)_x$-CHO oder z.B. $-(CH_2)_x$-Br, X = 0 bis 5) in Analogie zu den oben beschriebenen Methoden zugänglich sind.

Die Verbindungen der Formel I können weiterhin durch Kreuzkopplungen nach DOS 36 08 502, DOS 36 32 410 oder DOS 37 36 489 oder durch Wolff-Kishner-Reduktion entsprechender Methylenketone hergestellt werden, die ihrerseits leicht durch Friedel-Crafts-Acylierungen aus den entsprechenden Arylessigsäurechloriden und entsprechenden Benzol- oder Biphenyl-Vorstufen zugänglich sind.

Besonders bevorzugte Synthesevarianten der bevorzugten Verbindungen der Teilformel Ia, worin m = 1 und $L^1$ = $L^2$ = H, sind im folgenden Schema angegeben:

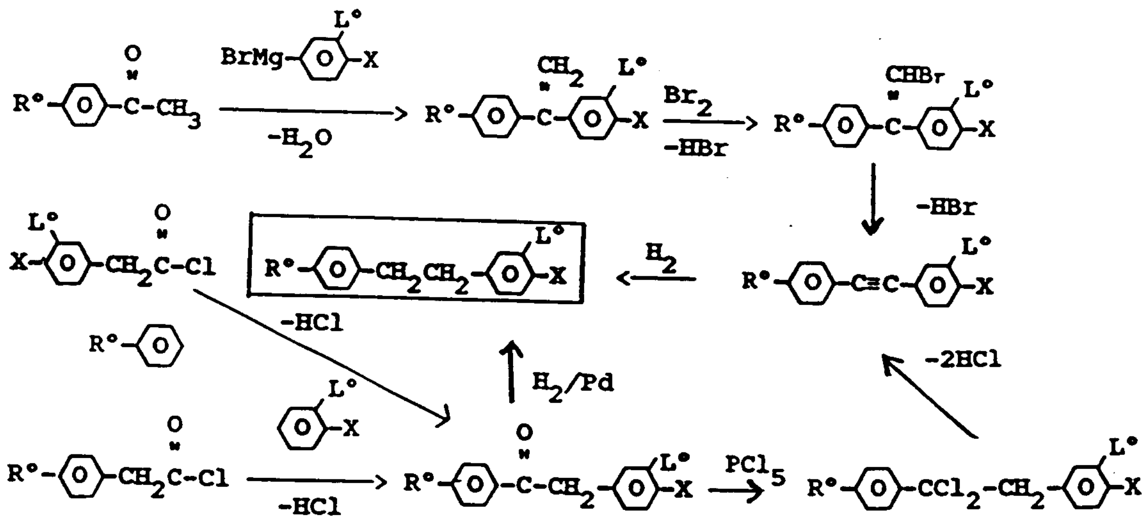


R° = 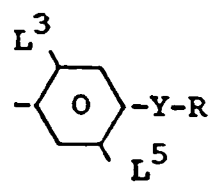

L° = H oder F

Vorzugsweise ist $L^3$ = F, $L^5$ = H,

Y = 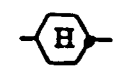

oder Einfachbindung, R = n-Alkyl, X = F, Cl, $CF_3$ oder $OCF_3$. L° ist vorzugsweise H.

Besonders bevorzugt sind die Verbindungen der Formeln I31 bis 36, I18, I19, I21 und I22. X ist hier vorzugsweise CN, F, Cl, $CF_3$, $OCF_3$ oder $OCHF_2$, insbesondere bevorzugt CN, F oder Cl. Y ist vorzugsweise trans-1,4-Cyclohexylen (insbesondere in I22) oder eine Einfachbindung.

Weitere besonders bevorzugte erfindungsgemäße Verbindungen sind in den beiden folgenden Tabellen aufgelistet Cy = trans-1,4-Cyclohexylen, Ph = 1,4-Phenylen,

PhF = 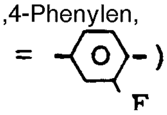):

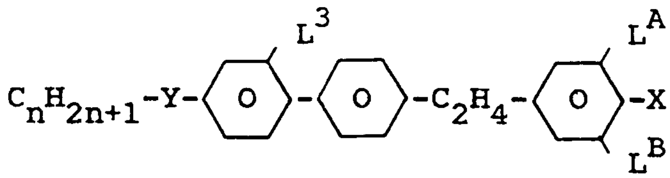

| n | Y | $L^3$ | $L^A$ | $L^B$ | X |
|---|---|---|---|---|---|
| 3 | - | F | H | H | CN |
| 5 | - | F | H | H | CN |
| 3 | - | F | F | H | CN |
| 2 | - | F | H | H | Cl |
| 3 | - | F | H | H | Cl |
| 3 | - | F | H | H | CN |
| 3 | Cy | H | F | H | CN |
| 5 | Cy | H | F | H | CN |
| 3 | Cy | H | F | F | CN |
| 5 | Cy | H | F | F | CN |
| 3 | - | H | H | H | Cl |
| 5 | - | H | H | H | Cl |
| 3 | - | H | F | H | Cl |
| 5 | - | H | F | H | Cl |
| 3 | Cy | F | H | H | CN |
| 5 | Cy | F | H | H | CN |

| n | Y | $L^3$ | $L^A$ | $L^B$ | X |
|---|---|---|---|---|---|
| 3 | Cy | F | F | H | CN |
| 5 | Cy | F | F | H | CN |
| 3 | Cy | F | F | H | F |

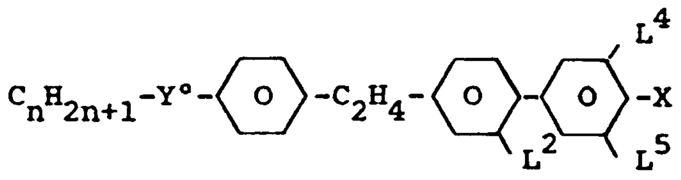

| n | Y° | $L^2$ | $L^4$ | $L^5$ | X |
|---|---|---|---|---|---|
| 3 | Cy | H | F | H | CN |
| 5 | Cy | H | F | H | CN |
| 3 | Cy | H | F | F | CN |
| 5 | Cy | H | F | F | CN |
| 3 | Ph | H | F | H | CN |
| 5 | Ph | H | F | H | CN |
| 3 | Ph | H | F | F | CN |
| 5 | Ph | H | F | F | CN |
| 3 | Ph | F | H | H | CN |
| 5 | Ph | F | H | H | CN |
| 3 | PhF | H | H | H | CN |
| 3 | Ph | F | H | H | CN |
| 3 | - | F | H | H | Cl |
| 3 | - | F | H | H | F |
| 3 | - | F | H | H | $CF_3$ |
| 3 | - | F | H | H | $OCF_3$ |
| 3 | - | F | H | H | $OCHF_2$ |
| 5 | - | F | H | H | Cl |
| 5 | - | F | H | H | F |

| n | Y° | $L^2$ | $L^4$ | $L^5$ | X |
|---|---|---|---|---|---|
| 5 | - | F | H | H | $CF_3$ |
| 5 | - | F | H | H | $OCF_3$ |
| 5 | - | F | H | H | $OCHF_2$ |
| 2 | - | F | H | H | Cl |
| 2 | - | F | H | H | F |
| 2 | - | F | H | H | $CF_3$ |
| 2 | - | F | H | H | $OCF_3$ |
| 2 | - | F | H | H | $OCHF_2$ |
| 4 | - | F | H | H | Cl |
| 4 | - | F | H | H | F |
| 4 | - | F | H | H | $CF_3$ |
| 4 | - | F | H | H | $OCF_3$ |
| 4 | - | F | H | H | $OCHF_2$ |
| 3 | - | H | F | H | Cl |
| 5 | - | H | F | H | Cl |
| 3 | - | H | F | H | F |
| 5 | - | H | F | H | F |
| 2 | - | H | H | H | F |
| 3 | - | H | H | H | F |
| 4 | - | H | H | H | F |
| 5 | - | H | H | H | F |
| 2 | - | H | H | H | Cl |
| 3 | - | H | H | H | Cl |
| 4 | - | H | H | H | Cl |
| 5 | - | H | H | H | Cl |
| 2 | - | H | F | H | F |
| 4 | - | H | F | H | F |

Weitere Synthesemöglichkeiten besonders bevorzugter Verbindungen sind in den folgenden Schemata A bis D angegeben (Y ist vorzugsweise von der Einfachbindung verschieden falls das Molekül eine 3-Fluor-4-cyanphenylgruppe enthält):

Schema A:

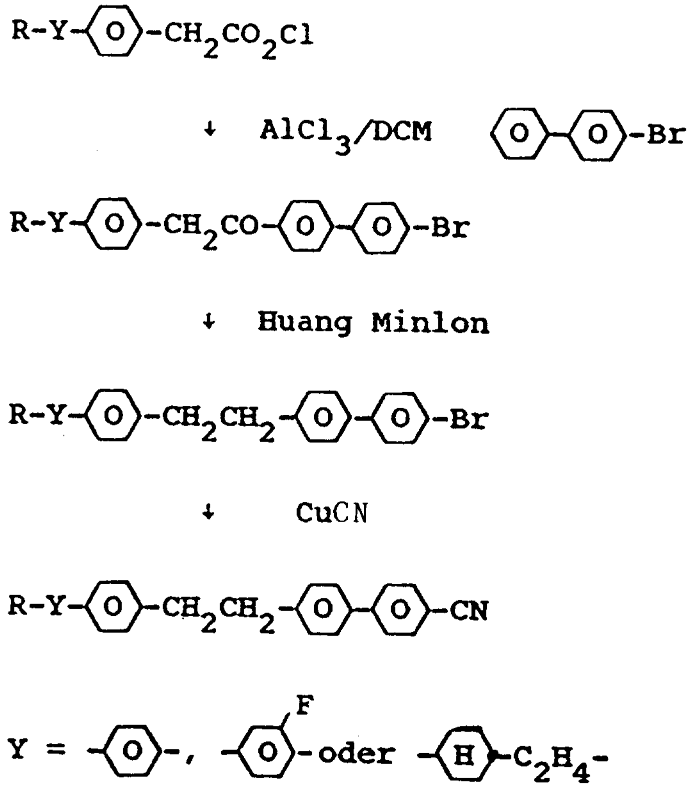


R = n-Alkyl mit bis zu 10 C-Atomen

Schema B:

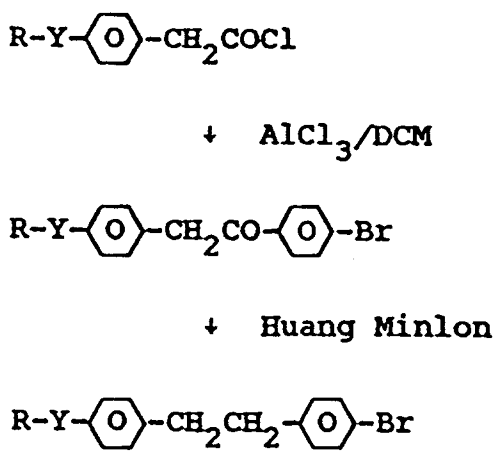

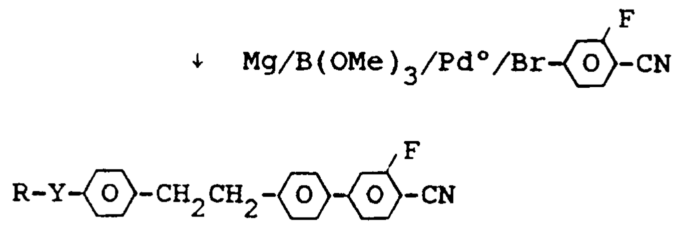


Y = 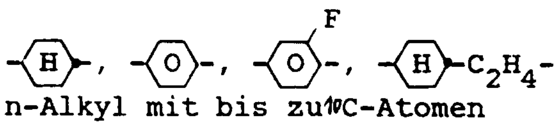 oder Einfachbindung

R = n-Alkyl mit bis zu 10 C-Atomen

Schema C:

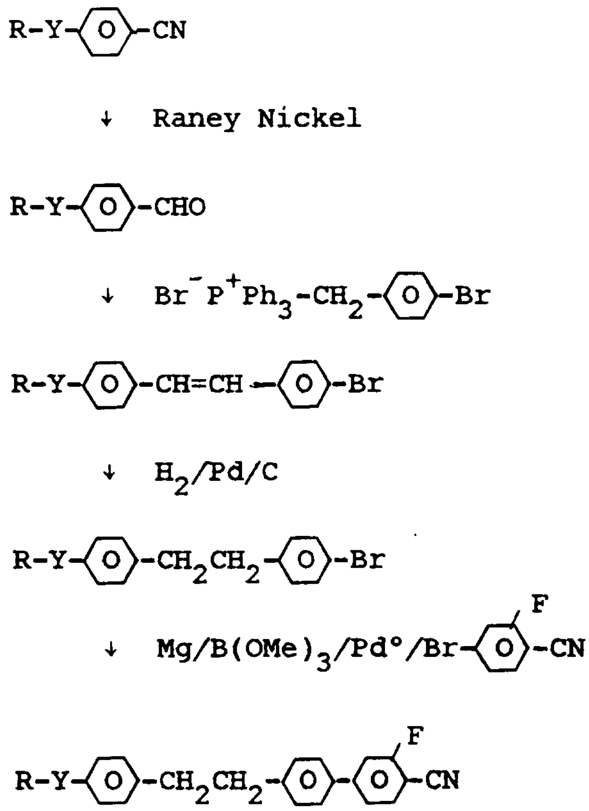


Y = 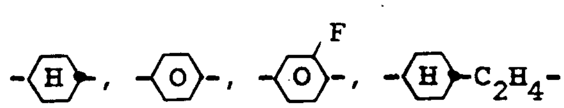 oder Einfachbindung

R = n-Alkyl mit bis zu 10 C-Atomen

Schema D:

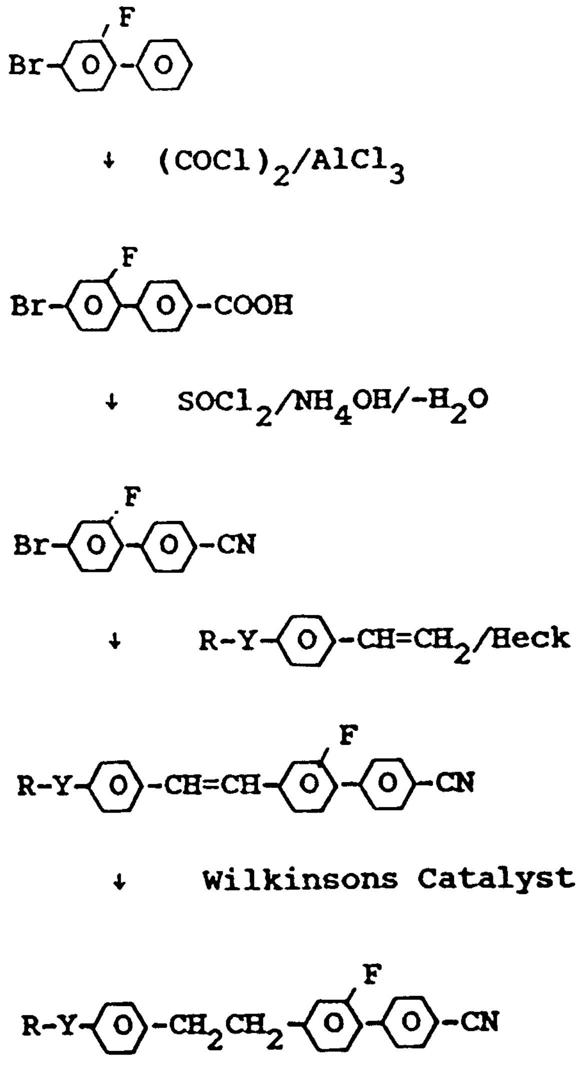


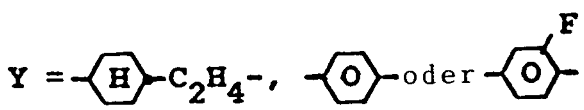

R = n-Alkyl mit bis zu 10 C-Atomen

Die erfindungsgmäßen Verbindungen mit X = CN eignen sich besonders als Komponenten von LC-Gemischen für PDLC (polymer dispersed liquid crystals) oder PN (polymer network).

Besonders bevorzugt sind vierkernige CN-Verbindungen (m = 2 oder

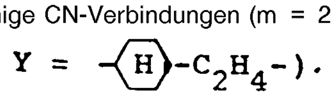

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen weitere 3 bis 20 Bestandteile. Die anderen Bestandteile werden vorzugsweise ausgewählt aus nematischen oder nematogenen (monotropen oder isotropen) Substanzen, insbesondere Substanzen aus

den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexybiphenyle, Phenyl- oder Cyclohexylpyrimidine, Phenyl oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch fluoriert sein. Die wichtigsten als weitere Bestandteile erfindungsgemämßer Medien in Frage kommenden Verbindungen lassen sich durch die Formeln 1, 2, 3, 4 und 5 charakterisieren:

R'-L-E-R'' 1

R'-L-COO-E-R'' 2

R'-L-OOC-E-R'' 3

R'-L-$CH_2CH_2$-E-R'' 4

R'-L-C≡C-E-R'' 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc, -Cyc-Cyc-, Pyr, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten auzgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, G-Phe- und -G-Cyc-.

R' und R'' bedeuten in den Verbindungen der Teilformeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind R' und R'' voneinander verschieden, wobei einer dieser Reste meist Alkyl oder Alkenyl ist. In den Verbindungen der Teilformeln 1b, 2b, 3b, 4b und 5b bedeutet R'' -CN, -$CF_3$, F, Cl oder -NCs; R hat dabei die bei den Verbindungen der Teilformeln 1a bis 5a angegebene Bedeutung und ist vorzugsweise Alkyl oder Alkenyl. Aber auch andere Varianten der vorgesehenen Substutienten in den Verbindungen der Formeln 1, 2, 3, 4 und 5 sind gebräuchlich. Viele solcher Substanzen oder auch Gemische davon sind im Handel erhältlich. Alle dieser Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:

Gruppe 1: 20 bis 90 %, insbesondere 30 bis 90 %,

Gruppe 2: 10 bis 80 %, insbesondere 10 bis 50 %,

wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise drei,

vier oder fünf erfindungsgemäße Verbindungen.

Die Herstellung der erfindungsgemäßen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vg. Z.B. I. Haller et al., Mol. Cryst. Liq. Cryst. Band 24, Seiten 249-258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Beispiel 1

Eine Lösung von 9,5 g 1-(p-Fluorphenyl)-2-(4'-n-pentylbiphenyl-4-yl)-ethen [erhalten durch Heckkopplung von 4-Fluorstyrol mit 4-Brom-4'-n-pentylbiphenyl] in 100 ml Tetrahydrofuran wird in Gegenwart von Pd/C bis zur Beendigung der H-Aufnahme hydriert Nach üblicher Aufarbeitung erhält man 1-(p-Fluorphenyl)-2-(4'-n-pentylbiphenyl-4-yl)-ethan, F. 60°, K. 109°.

Analog werden hergestellt:

1-(p-Fluorphenyl)-2-(4'-n-propylbiphenyl-4-yl)-ethan, F. 104°
1-(p-Chlorphenyl)-2-(4'-n-propylbiphenyl-4-yl)-ethan
1-(3-Fluor-4-chlorphenyl)-2-(4'-n-propylbiphenyl-4-yl)-ethan
1-(p-Chlorphenyl)-2-(4'-n-propyl-2'-fluorbiphenyl-4-yl)-ethan, F. 63,6°
1-(p-Trifluormethylphenyl)-2-(4'-ethylbiphenyl-4-yl)-ethan
1-(p-Trifluormethylphenyl)-2-(4'-n-propylbiphenyl-4-yl)-ethan, F. 128°
1-(p-Trifluormethylphenyl)-2-(4'-n-butylbiphenyl-4-yl)-ethan
1-(p-Trifluormethylphenyl)-2-(4'-n-pentylbiphenyl-4-yl)-ethan, F. 85°, K. 126°
1-(p-Trifluormethylphenyl)-2-(4'-n-propyl-2'-fluorbiphenyl-4-yl)-ethan
1-(p-Trifluormethylphenyl)-2-(4'-n-pentyl-2'-fluorbiphenyl-4-yl)-ethan
1-(p-Trifluormethoxyphenyl)-2-(4'-ethylbiphenyl-4-yl)-ethan
1-(p-Trifluormethoxyphenyl)-2-(4'-n-propylbiphenyl-4-yl)-ethan, F. 76°, K. 141°
1-(p-Trifluormethoxyphenyl)-2-(4'-n-butylbiphenyl-4-yl)-ethan,
1-(p-Trifluormethoxyphenyl)-2-(4'-n-pentylbiphenyl-4-yl)-ethan, F. 62°, K. 136°
1-(p-Trifluormethoxyphenyl)-2-(4'-n-propyl-2'-fluorbiphenyl-4-yl)-ethan
1-(p-Trifluormethoxyphenyl)-2-(4'-n-pentyl-2'-fluorbiphenyl-4-yl)-ethan
1-(p-Difluormethoxyphenyl)-2-(4'-ethylbiphenyl-4-yl)-ethan
1-(p-Difluormethoxyphenyl)-2-(4'-n-propylbiphenyl-4-yl)-ethan
1-(p-Difluormethoxyphenyl)-2-(4'-n-butylbiphenyl-4-yl)-ethan
1-(p-Difluormethoxyphenyl)-2-(4'-n-propyl-2'-fluorbi-phenyl-4-yl)-ethan
1-(p-Difluormethoxyphenyl)-2-(4'-n-pentyl-2'-fluorbiphenyl-4-yl)-ethan
1-(p-Chlorphenyl)-2-(4'-n-butyl-2'-fluorbiphenyl-4-yl)-ethan
1-(p-Chlorphenyl)-2-(4'-n-pentyl-2'-fluorbiphenyl-4-yl)-ethan, F. 49°, K. 82°
1-(p-Chlorphenyl)-2-(4'-n-heptyl-2'-fluorbiphenyl-4-yl)-ethan
1-(p-Chlorphenyl)-2-(4'-n-ethyl-2'-fluorbiphenyl-4-yl)-ethan, F. 62,4°
1-(p-Fluorphenyl)-2-(4'-ethyl-2'-fluorbiphenyl-4-yl)-ethan
1-(p-Fluorphenyl)-2-(4'-n-propyl-2'-fluorbiphenyl-4-yl)-ethan
1-(p-Fluorphenyl)-2-(4'-n-butyl-2'-fluorbiphenyl-4-yl)-ethan
1-(p-Fluorphenyl)-2-(4'-n-pentyl-2'-fluorbiphenyl-4-yl)-ethan, F. 35°, K 54°
1-(p-Fluorphenyl)-2-(4'-n-heptyl-2'-fluorbiphenyl-4-yl)-ethan
1-(p-Fluorphenyl)-2-[4'-(trans-4-ethylcyclohexyl)-2'-fluorbiphenyl-4-yl)-ethan

1-(p-Fluorphenyl)-2-[4'-(trans-4-n-propylcycloxexyl)-2'-fluorbiphenyl-4-yl)-ethan
1-(p-Fluorphenyl)-2-[4'-(trans-4-n-butylcyclohexyl)-2'-fluorbiphenyl-4-yl)-ethan
1-(p-Fluorphenyl)-2-[4'-(trans-4-n-pentylcyclohexyl)-2'-fluorbiphenyl-4-yl)-ethan, F. 68°, K. 206°
1-(p-Fluorphenyl)-2-[4'-(trans-4-n-heptylcyclohexyl)-2'-fluorbiphenyl-4-yl)-ethan
1-(p-Chlorphenyl-2-[4'-(trans-4-ethylcyclohexyl)-2'-fluorbiphenyl-4-yl)-ethan
1-(p-Chlorphenyl-2-[4'-(trans-4-n-propylcyclohexyl)-2'-fluorbiphenyl-4-yl)-ethan
1-(p-Chlorphenyl-2-[4'-(trans-4-n-butylcyclohexyl)-2'-fluorbiphenyl-4-yl)-ethan
1-(p-Chlorphenyl-2-[4'-(trans-4-n-pentylcyclohexyl)-2'-fluorbiphenyl-4-yl)-ethan
1-(p-Chlorphenyl-2-[4'-(trans-4-n-heptylcyclohexyl)-2'-fluorbiphenyl-4-yl)-ethan
1-(p-Fluorphenyl)-2-(4'-n-butoxybiphenyl-4-yl)-ethan, F. 102°, K. 145°
1-(p-Fluorphenyl)-2-(4'-n-propoxybiphenyl-4-yl)-ethan
1-(p-Fluorphenyl)-2-(4'-n-ethoxybiphenyl-4-yl)-ethan
1-(3,5-Difluoryhenyl)-2-(4'-ethylbiphenyl-4-yl)-ethan
1-(3,5-Difluorphenyl)-2-(4'-n-propylbiphenyl-4-yl)-ethan
1-(3,5-Difluorphenyl)-2-(4'-n-butylbiphenyl-4-yl)-ethan
1-(3,5-Difluoryhenyl)-2-(4'-n-pentylbiphenyl-4-yl)-ethan, F. 59°
1-(p-Chlorphenyl)-2-(4'-ethylbiphenyl-4-yl)-ethan
1-(p-Chlorphenyl)-2-(4'-n-propylbiphenyl-4-yl)-ethan
1-(p-Chlorphenyl)-2-(4'-n-butylbiphenyl-4-yl)-ethan
1-(p-Chlorphenyl)-2-(4'-n-pentylbiphenyl-4-yl)-ethan, F. 129°

Beispiel 2

Eine Lösung von 10,6 g 1-(p-n-Propylphenyl)-2-(4'-fluorbiphenyl-4-yl)-ethen [erhalten durch Heckkopplung von 4-Propylstyrol mit 4-Brom-4'-n-fluorbiphenyl] in 200 ml Tetrahydrofuran wird in Gegenwart von Pd/C bis zur Beendigung der H-Aufnahme hydriert. Nach üblicher Aufarbeitung erhält man 1-(p-Propylphenyl)-2-(4'-fluorbiphenyl-4-yl)-ethan, F. 97°.

1-(p-Ethoxyphenyl)-2-(4'-trifluormethoxybiphenyl-4-yl)-ethan, F. 165°
1-(p-Ethylphenyl)-2-(4'-trifluormethoxybiphenyl-4-yl)-ethan
1-(p-n-Propylphenyl)-2-(4'-trifluormethoxybiphenyl-4-yl)-ethan, F. 97°, K. 137°
1-(p-Butylphenyl)-2-(4'-trifluormethoxybiphenyl-4-yl)-ethan
1-(p-Pentylphenyl)-2-(4'-trifluormethoxybiphenyl-4-yl)-ethan
1-(p-n-Propylphenyl)-2-(4'-chlor-3'-fluorbiphenyl-4-yl)-ethan, F, 60°, K. 65°
1-(p-n-Butylphenyl)-2-(4'-chlor-3'-fluorbiphenyl-4-yl)-ethan
1-(p-n-Pentylphenyl)-2-(4'-chlor-3'-fluorbiphenyl-4-yl)-ethan
1-(p-Ethoxyphenyl)-2-(4'-difluormethoxybiphenyl-4-yl)-ethan, F. 153°
1-(p-Ethylphenyl)-2-(4'-difluormethoxybiphenyl-4-yl)-ethan
1-(p-n-Propylphenyl)-2-(4'-difluormethoxybiphenyl-4-yl)-ethan, F. 131°
1-(p-n-Butylphenyl)-2-(4'-difluormethoxybiphenyl-4-yl)-ethan
1-(p-n-Pentylphenyl)-2-(4'-difluormethoxybiphenyl-4-yl)-ethan
1-(p-Ethoxyphenyl)-2-(4'-fluorbiphenyl-4-yl)-ethan, F. 121°, K. 130°
1-(p-Ethylphenyl)-2-(4'-fluorbiphenyl-4-yl)-ethan
1-(p-n-Propylphenyl)-2-(4'-fluorbiphenyl-4-yl)-ethan
1-(p-n-Butylphenyl)-2-(4'-fluorbiphenyl-4-yl)-ethan
1-(p-n-Pentylphenyl)-2-(4'-fluorbiphenyl-4-yl)-ethan
1-(p-Ethylphenyl)-2-(2',4'-difluorbiphenyl-4-yl)-ethan
1-(p-n-Propylphenyl)-2-(2',4'-difluorbiphenyl-4-yl)-ethan
1-(p-n-Butylphenyl)-2-(2',4'-difluorbiphenyl-4-yl)-ethan
1-(p-n-Pentylphenyl)-2-(2',4'-difluorbiphenyl-4-yl)-ethan, F. 40°, K. 50°
1-(p-Ethylphenyl)-2-(3',4'-difluorbiphenyl-4-yl)-ethan
1-(p-n-Propylphenyl)-2-(3',4'-difluorbiphenyl-4-yl)-ethan
1-(p-n-Butylphenyl)-2-(3',4'-difluorbiphenyl-4-yl)-ethan
1-(p-n-Pentylphenyl)-2-(3',4'-difluorbiphenyl-4-yl)-ethan, F. 46°
1-(p-Ethylphenyl)-2-(3'-fluor-4'-chlorbiphenyl-4-yl)-ethan
1-(p-n-Propylphenyl)-2-(3'-fluor-4'-chlorbiphenyl-4-yl)-ethan
1-(p-n-Butylphenyl)-2-(3'-fluor-4'-chlorbiphenyl-4-yl)-ethan
1-(p-n-Pentylphenyl)-2-(3'-fluor-4'-chlorbiphenyl-4-yl)-ethan
1-(2-Fluor-4-n-propylphenyl)-2-(4'-fluorbiphenyl-4-yl)-ethan

1-(2-Fluor-4-n-pentylphenyl)-2-(4'-fluorbiphenyl-4-yl)-ethan
1-(2-Fluor-4-n-propylphenyl)-2-(4'-chlorbiphenyl-4-yl)-ethan
1-(2-Fluor-4-n-pentylphenyl)-2-(4'-chlorbiphenyl-4-yl)-ethan
1-(2-Fluor-4-n-propylphenyl)-2-(4'-cyanbiphenyl-4-yl)-ethan
1-(2-Fluor-4-n-pentylphenyl)-2-(4'-cyanbiphenyl-4-yl)-ethan
1-(2-Fluor-4-n-propylphenyl)-2-(4'-trifluormethylbiphenyl-4-yl)-ethan
1-(2-Fluor-4-n-pentylphenyl)-2-(4'-trifluormethylbiphenyl-4-yl)-ethan
1-(2-Fluor-4-n-propylphenyl)-2-(4'-trifluormethoxybiphenyl-4-yl)-ethan
1-(2-Fluor-4-n-pentylphenyl)-2-(4'-trifluormethoxybiphenyl-4-yl)-ethan
1-(2-Fluor-4-n-propylphenyl)-2-(4'-difluormethoxybiphenyl-4-yl)-ethan
1-(2-Fluor-4-n-pentylphenyl)-2-(4'-difluormethoxybiphenyl-4-yl)-ethan
1-(p-n-Propylphenyl)-2-(4'-trifluormethyl-2'-fluorbiphenyl-4-yl)-ethan
1-(p-n-Propylphenyl)-2-(4'-trifluormethoxy-2'-fluorbiphenyl-4-yl)-ethan
1-(p-n-Propylphenyl)-2-(2',4'-difluorobiphenyl-4-yl)-ethan

**Patentansprüche**

**1.** Biphenylylethane der Formel I

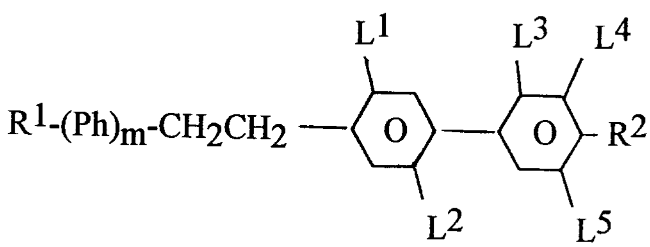

I

worin einer der Reste $R^1$ und $R^2$ X und der andere Rest $R^1$ bzw. $R^2$ R-Y- bedeutet, wobei

| | |
|---|---|
| X | H, F, Cl, $-CF_3$, $-OCHF_2$, $-OCF_3$, -CN, -NCS oder $-NO_2$, |
| R | Alkyl, Fluoralkyl, Alkenyl oder Oxaalkyl und |
| Y | O, S, -CO-O-, -O-CO-, -O-CO-O-, eine Einfachbindung, oder - im Falle m = 1 - auch trans-1,4-Cyclohexylen oder 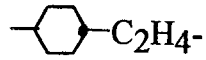 bedeutet, |
| m | 1 oder 2, |
| Ph | jeweils gleiche oder verschiedene Reste ausgewählt aus der Gruppe bestehend aus 1,4-Phenylen, 2-Fluor-1,4-phenylen, 3-Fluor-1,4-phenylen und 3,5-Difluor-1,4-phenylen, |
| $L^3$ und $L^4$ | jeweils H, oder einer dieser Reste auch F, |
| $L^1$, $L^2$ und $L^5$ | jeweils H oder F bedeutet, mit den Maßgaben, daß |

(a) im Falle

X = CN Y 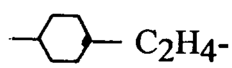

bedeutet und/oder m = 2 und/oder $L^1$ Fluor bedeutet und/oder Ph-CN 2-Fluor-4-cyanphenyl oder 3,5-Difluor-4-cyanphenyl bedeutet und

(b) im Falle

$R^1$ = -NCS Y 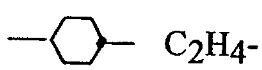

bedeutet und/oder m = 2 und/oder einer der Reste $L^1$, $L^2$ und $L^3$ Fluor bedeutet, sowie Biphenylylethane der Formel I36

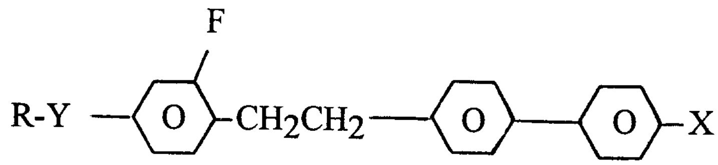 I36

worin R und Y die oben angegebene Bedeutung haben und X -CN oder -NCS bedeutet.

**2.** Biphenylylethane der Formel Ia,

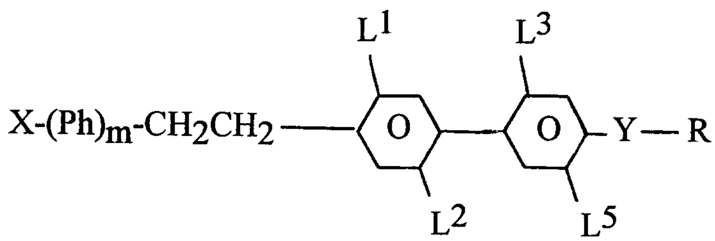 Ia

worin

| | |
|---|---|
| R | Alkyl, Fluoralkyl, Alkenyl oder Oxaalkyl, |
| m | 1 oder 2, |
| Y | O, S, CO-O, O-CO, O-CO-O, eine Einfachbindung oder - im Falle m = 1 - auch trans-1,4-Cyclohexylen, |
| Ph | jeweils gleiche oder verschiedene Reste ausgewählt aus der Gruppe bestehend aus 1,4-Phenylen, 2-Fluor-1,4-phenylen, 3-Fluor-1,4-phenylen und 3,5-Difluor-1,4-phenylen, |
| $L^1$, $L^2$ $L^3$ und $L^5$ | jeweils H oder F, |
| X | F, Cl, $-CF_3$, -CN, - $OCF_3$, $-OCHF_2$, -NCS oder $-NO_2$ bedeutet, mit der Maßgabe, daß |

a) im Falle X = -CN Y trans-1,4-Cyclohexylen oder m = 2 und/oder Ph-X 2-Fluor-4-cyanphenyl, 3-Fluor-4-cyanphenyl oder 3,5-Difluor-4-cyanphenyl und/oder einer oder zwei der Reste $L^1$, $L^2$, $L^3$ und $L^5$ F bedeutet, und

b) im Falle X = NCS m = 2 und/oder Ph-X 2-Fluor-4-isothiocyanatophenyl,3-Fluor-4-isothiocyanatophenyl und/oder einer oder zwei der Reste $L^1$, $L^2$, $L^3$ und $L^5$ F und/oder -Y-R geradkettiges Alkyl mit bis zu 3 C-Atomen bedeutet.

**3.** Biphenylylethane der Formel Ib,

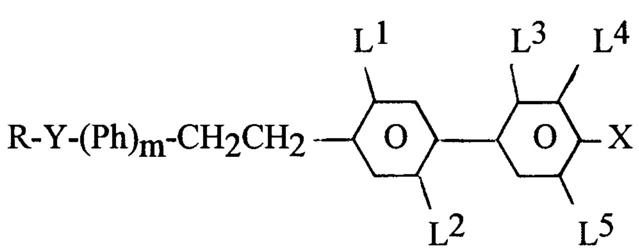 Ib

worin

| | |
|---|---|
| R | Alkyl, Fluoralkyl, Alkenyl oder Oxaalkyl, |
| m | 1 oder 2, |
| Y | O, S, CO-O, O-CO, O-CO-O, eine Einfachbindung oder - im Falle m = 1 - auch trans-1,4-Cyclohexylen, |
| Ph | jeweils gleiche oder verschiedene Reste ausgewählt aus der Gruppe bestehend aus 1,4-Phenylen, 2-Fluor-1,4-phenylen, 3-Fluor-1,4-phenylen und 3,5-Difluor-1,4-phenylen, |

| | |
|---|---|
| $L^1$, $L^3$ und $L^4$ | jeweils H, oder einer dieser Reste auch F, |
| $L^2$ und $L^5$ | jeweils H oder F, und |
| X | F, Cl, $-CF_3$, -CN, $-OCF_3$, -NCS oder $-NO_2$ bedeutet, mit der Maßgabe, daß |

im Falle X = F, Cl, -CN oder $-CF_3$ m = 2 und/oder $L^4$ und/oder $L^5$ Fluor (falls X = Halogen oder $-CF_3$) bzw. $L^4$ und $L^5$ oder $L^1$ (falls X = CN) Fluor bedeutet.

**4.** Verbindungen nach Anspruch 2, gekennzeichnet durch die Teilformel Ia1

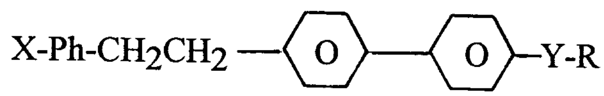

Ia1

worin X, Ph und R die angegebene Bedeutung haben und Y 0 oder eine Einfachbindung bedeutet.

**5.** Verbindungen nach Anspruch 3, gekennzeichnet durch die Teilformel Ib1

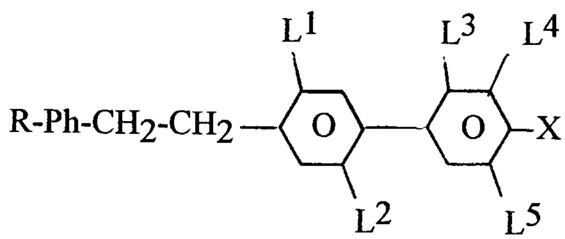

Ib1

worin R, Ph, $L^1$, $L^2$, $L^3$, $L^4$, $L^5$ und X die angegebene Bedeutung haben.

**6.** Verbindungen und Anspruch 1, dadurch gekennzeichnet, daß X F, Cl, $-CF_3$, $-OCF_3$ oder $-OCHF_2$ bedeutet.

**7.** Verbindungen der Formel Ia4

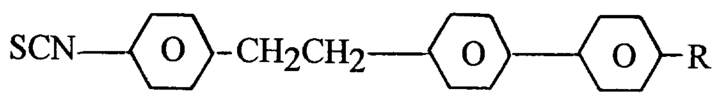

Ia4

worin R Methyl, Ethyl oder n-Propyl bedeutet.

**8.** Verbindungen nach Anspruch 2, gekennzeichnet durch die Formel

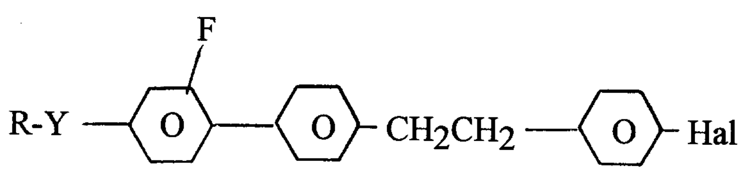

I2

worin Hal F oder Cl bedeutet und R und Y die angegebene Bedeutung haben.

**9.** Verbindungen nach Anspruch 2, gekennzeichnet durch die Formeln

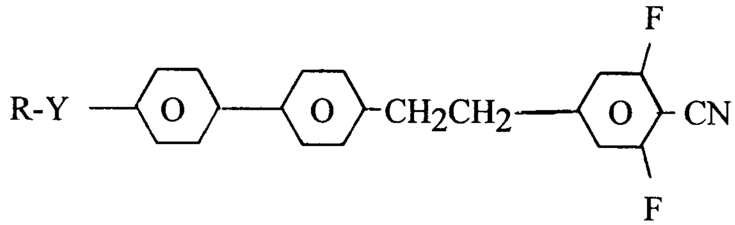

I10a

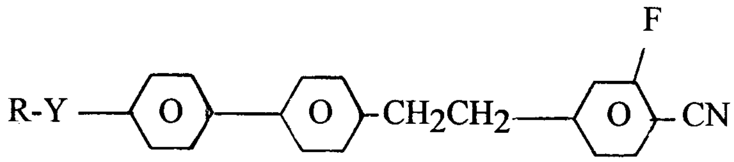

I10b

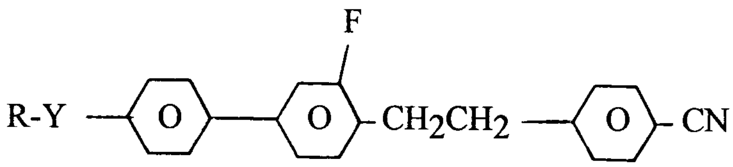

I11a

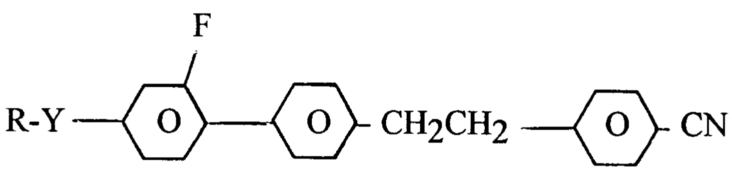

I11b

worin R und Y die angegebene Bedeutung haben.

**10.** Verbindungen nach Anspruch 1, gekennzeichnet durch die Formeln

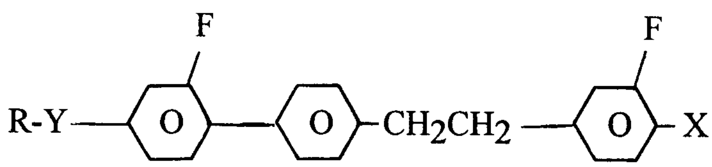

I13

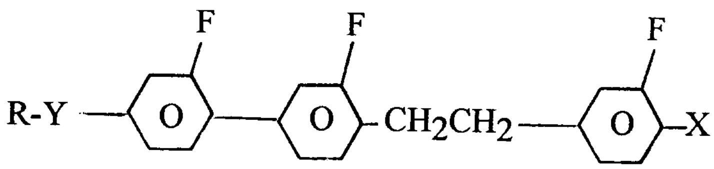

I14

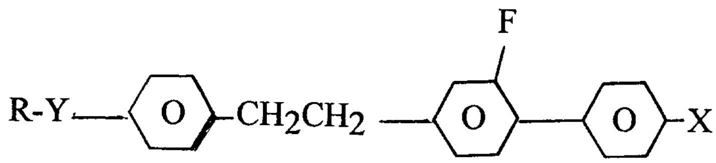

I18

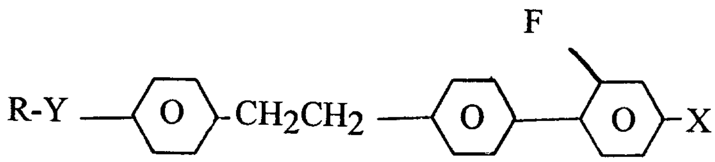

I19

worin R, Y und X die angegebenen Bedeutungen haben.

**11.** Verbindungen nach Anspruch 3, gekennzeichnet durch die Formel

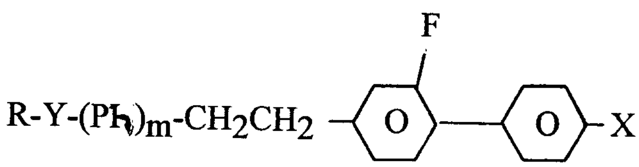

V

worin X F, Cl, $CF_3$, $OCF_3$ oder $OCHF_2$ bedeutet und R, Y, Ph und m die angegebene Bedeutung haben.

**12.** Verbindungen nach Anspruch 1, dadurch gekennzeichnet, daß $R^1$-$(Ph)_m$- und $L^1$ bis $L^5$ die folgenden Bedeutungen haben:

| $R^1$-(Ph)$_m$- | $L^1$ | $L^2$ | $L^3$ | $L^4$ | $L^5$ |
|---|---|---|---|---|---|
| R-Y-(Ph)$_m$- | H | F | H | H | H |
| R-Y-(Ph)$_m$- | H | H | H | F | H |
| R-Y-(Ph)$_m$- | H | H | H | F | F |
| R-Y-(Ph)$_m$- | H | F | H | F | H |
| R-Y-(Ph)$_m$- | H | F | H | F | F |
| R-Y 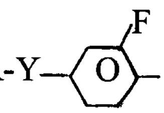 | H | H | H | H | H |
| R-Y 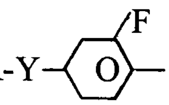 | H | F | H | H | H |
| X-(Ph)$_m$ | H | H | F | H | H |
| X-(Ph)$_m$ | F | H | H | H | H |
| X-(Ph)$_m$ | F | H | F | H | H |
| X 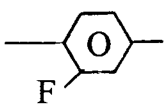 | H | H | H | H | H |
| X 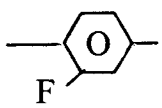 | H | H | F | H | H |

**13.** Verwendung der Verbindungen der Formel I nach Anspruch 1 als Komponenten flüssigkristalliner Phasen.

**14.** Flüssigkristalline Phase mit mindestens zwei flüssigkristallinen Komponenten, dadurch gekennzeichnet, daß sie mindestens eine Verbindung der Formel I enthält.

**15.** Flüssigkristallanzeigeelement, dadurch gekennzeichnet, daß es eine flüssigkristalline Phase nach Anspruch 14 enthält.

## Claims

1. Biphenylylethanes of the formula I

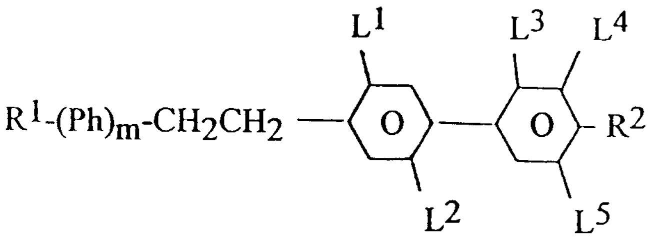

I

in which one of the radicals $R^1$ and $R^2$ is X and the other radical $R^1$ or $R^2$ is R-Y- where

X is H, F, Cl, $-CF_3$, $-OCHF_2$, $-OCF_3$, -CN, -NCS or - $NO_2$,

R is alkyl, fluoroalkyl, alkenyl or oxaalkyl and

Y is O, S, -CO-O-, -O-CO-, -O-CO-O-, a single bond or - in the case where m = 1 - alternatively trans-1,4-cyclohexylene or

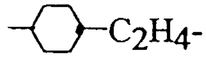

,

m is 1 or 2,

Ph is in each case identical or different radicals selected from the group comprising 1,4-phenylene, 2-fluoro-1,4-phenylene, 3-fluoro-1,4-phenylene and 3,5-difluoro-1,4-phenylene,

$L^3$ and $L^4$ are each H, or one of these radicals is alternatively F,

$L^1$, $L^2$ and $L^5$ are each H or F, with the provisos that

(a) in the case where X = CN, Y is

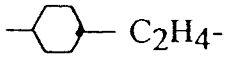

and/or m = 2 and/or $L^1$ is fluorine and/or Ph-CN is 2-fluoro-4-cyanophenyl or 3,5-difluoro-4-cyanophenyl and

(b) in the case where $R^1$ = -NCS,Y is

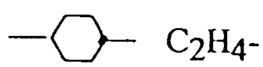

and/or m = 2 and/or one of the radicals $L^1$, $L^2$ and $L^3$ is fluorine,

and biphenylylethanes of formula I36

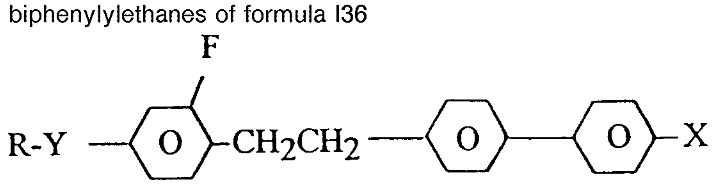

I36

in which R and Y are as defined above, and X is -CN or -NCS.

2. Biphenylylethanes of the formula Ia,

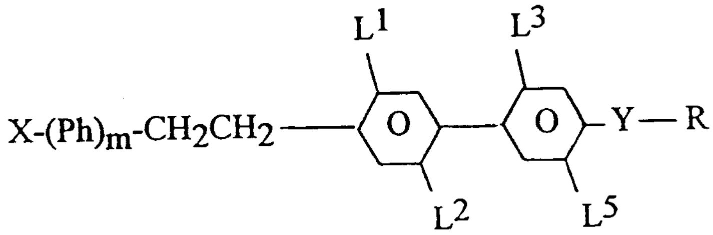

Ia

in which

| | |
|---|---|
| R | is alkyl, fluoroalkyl, alkenyl or oxaalkyl, |
| m | is 1 or 2, |
| Y | is O, S, CO-O, O-CO, O-CO-O, a single bond or - in the case where m = 1 - alternatively trans1,4-cyclohexylene, |
| Ph | is in each case identical or different radicals selected from the group comprising 1,4-phenylene, 2-fluoro-1,4-phenylene, 3-fluoro-1,4-phenylene and 3,5-difluoro-1,4-phenylene, |
| $L^1$, $L^2$, $L^3$ and $L^5$ | are each H or F, |
| X | is F, Cl, $-CF_3$, -CN, $-OCF_3$, $-OCHF_2$, -NCS or - $NO_2$, with the proviso that<br>a) in the case where X = -CN, Y is trans-1,4-cyclohexylene or m = 2 and/or Ph-X is 2-fluoro-4-cyanophenyl, 3-fluoro-4-cyanophenyl or 3,5-difluoro-4-cyanophenyl and/or one or two of the radicals $L^1$ , $L^2$, $L^3$ and $L^5$ is F, and<br>b) in the case where X = NCS, m = 2 and/or Ph-X is 2-fluoro-4-isothiocyanatophenyl, 3-fluoro-4-isothiocyanatophenyl and/or one or two of the radicals $L^1$, $L^2$, $L^3$ and $L^5$ is F and/or -Y-R is straight-chain alkyl having up to 3 C atoms, |

**3.** Biphenylylethanes of the formula Ib,

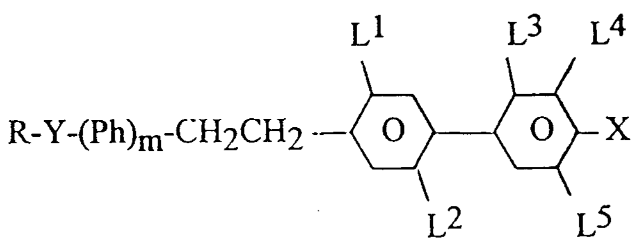

Ib

in which

| | |
|---|---|
| R | is alkyl, fluoroalkyl, alkenyl or oxaalkyl, |
| m | is 1 or 2, |
| Y | is O, S, CO-O, O-CO, O-CO-O, a single bond or - in the case where m = 1 - alternatively trans-1,4-cyclohexylene, |
| Ph | is in each case identical or different radicals selected from the group comprising 1,4-phenylene, 2-fluoro-1,4-phenylene, 3-fluoro-1,4-phenylene and 3,5-difluoro-1,4-phenylene, |
| $L^1$, $L^3$, and $L^4$ | are each H, or one of these radicals is alternatively F, |
| $L^2$ and $L^5$ | are each H or F, and |
| X | is F, Cl, $-CF_3$, -CN, $-OCF_3$, -NCS or $-NO_2$, with the proviso that, |

in the case where X = F, Cl, -CN or $-CF_3$, m = 2 and/or $L^4$ and/or $L^5$ is fluorine (if X = halogen or $-CF_3$) or $L^4$ and $L^5$ or $L^1$ (if X = CN) is fluorine.

**4.** Compounds according to Claim 2, characterized by the sub-formula Ia1

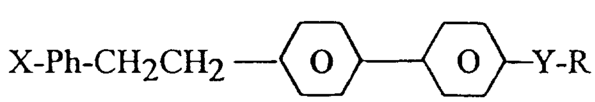

Ia1

in which X, Ph and R are as defined above, and Y is 0 [sic] or a single bond.

**5.** Compounds according to Claim 3, characterized by the sub-formula Ib1

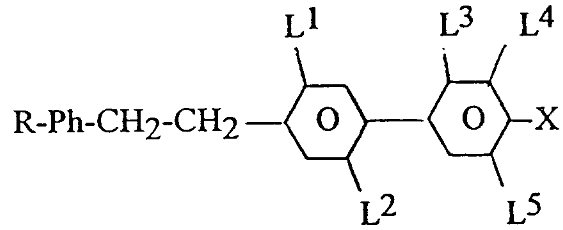

Ib1

in which R, Ph, $L^1$, $L^2$, $L^3$, $L^4$, $L^5$ and X are as defined above.

**6.** Compounds and [sic] Claim 1, characterized in that X is F, Cl, $-CF_3$, $-OCF_3$ or $-OCHF_2$.

**7.** Compounds of the formula Ia4

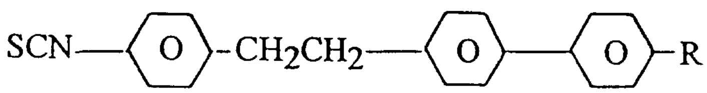

Ia4

in which R is methyl, ethyl or n-propyl.

**8.** Compounds according to Claim 2, characterized by the formula

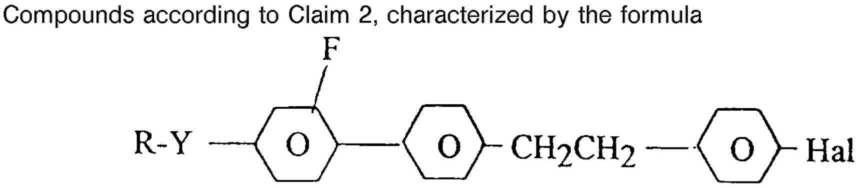

I2

in which Hal is F or Cl, and R and Y are as defined above.

**9.** Compounds according to Claim 2, characterized by the formulae

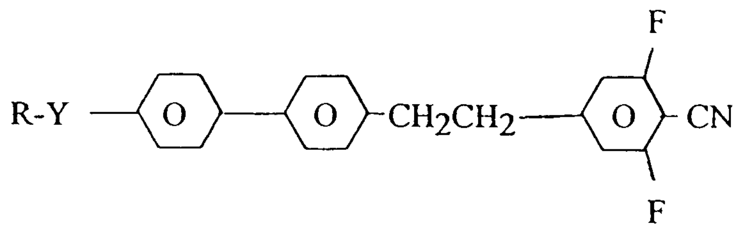

I10a

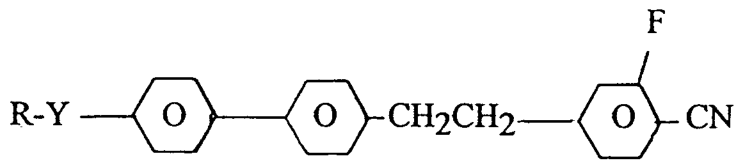

I10b

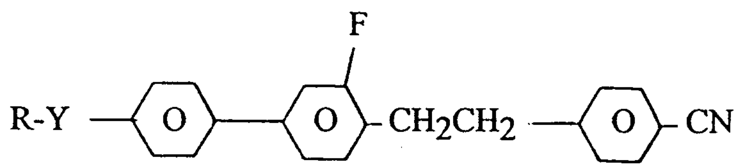

I11a

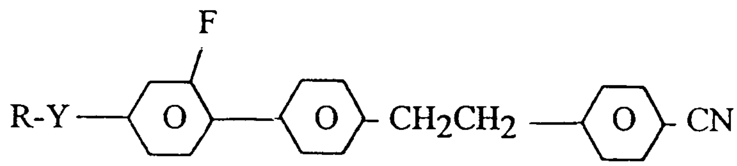

I11b

in which R and Y are as defined above.

**10.** Compounds according to Claim 1, characterized by the formulae

I13

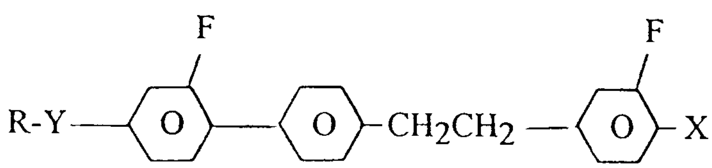

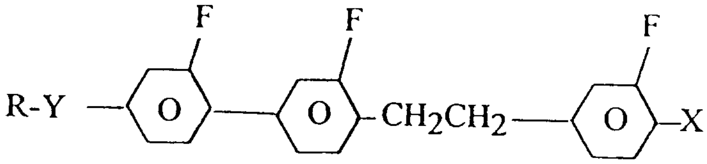

I14

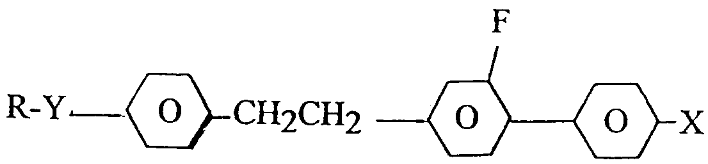

I18

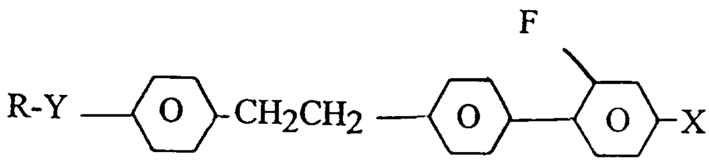

I19

in which R, Y and X are as defined above.

**11.** Compounds according to Claim 3, characterized by the formula

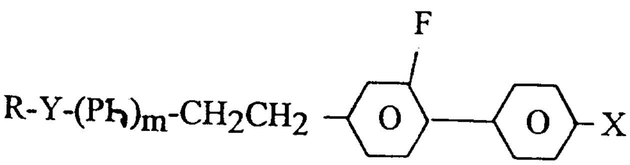

V

in which X is F, Cl, $CF_3$, $OCF_3$ or $OCHF_2$, and R, Y, Ph and m are as defined above.

**12.** Compounds according to Claim 1, characterized in that $R^1$-$(Ph)_{m}$- and $L^1$ to $L^5$ are as defined above:

| $R^1$-(Ph)$_m$- | $L^1$ | $L^2$ | $L^3$ | $L^4$ | $L^5$ |
|---|---|---|---|---|---|
| R-Y-(Ph)$_m$- | H | F | H | H | H |
| R-Y-(Ph)$_m$- | H | H | H | F | H |
| R-Y-(Ph)$_m$- | H | H | H | F | F |
| R-Y-(Ph)$_m$- | H | F | H | F | H |
| R-Y-(Ph)$_m$- | H | F | H | F | F |
| R-Y 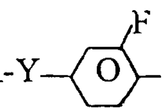 | H | H | H | H | H |
| R-Y 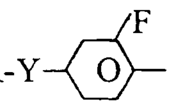 | H | F | H | H | H |
| X-(Ph)$_m$ | H | H | F | H | H |
| X-(Ph)$_m$ | F | H | H | H | H |
| X-(Ph)$_m$ | F | H | F | H | H |
| X 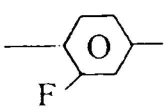 | H | H | H | H | H |
| X 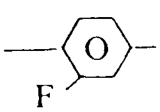 | H | H | F | H | H |

**13.** Use of the compounds of the formula I according to Claim 1 as components of liquid-crystalline phases.

**14.** Liquid-crystalline phase containing at least two liquid-crystalline components, characterized in that it contains at least one compound of the formula I.

**15.** Liquid-crystal display element, characterized in that it contains a liquid-crystalline phase according to Claim 14.

## Revendications

**1.** Diphénylyléthanes de formule I

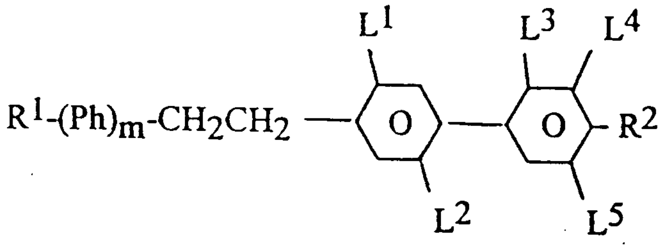 I

où un des restes $R^1$ et $R^2$ représente X et l'autre reste $R^1$ ou $R^2$ représente R-Y, où

X représente H, F, Cl, $-CF_3$, $-OCHF_2$, $-OCF_3$, -CN, -NCS ou - $NO_2$,

R représente un alkyle, un fluoroalkyle, un alcényle ou un oxa-alkyle et

Y représente O, S, -CO-O-, -O-CO-, -O-CO-O-, une liaison simple, ou, dans le cas où m = 1, également le trans-1,4-cyclohexylène ou

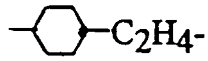 ,

m est 1 ou 2,

Ph représente chaque fois des restes identiques ou différents choisis dans le groupe constitué par le 1,4-phénylène, le 2-fluoro-1,4-phénylène, le 3-fluoro-1,4-phénylène et le 3,5-difluoro-1,4-phénylène,

$L^3$ et $L^4$ représentent à chaque fois H ou un de ces restes représente aussi F,

$L^1$, $L^2$ et $L^5$ représentent chaque fois H ou F, avec les conditions que

(a) dans le cas où X = CN, Y représente

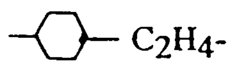

et/ou m = 2 et/ou $L^1$ représente le fluor et/ou Ph-CN représente le 2-fluoro-4-cyanophényle ou le 3,5-difluoro-4-cyanophényle, et

(b) dans le cas où $R^1$ = -NCS, Y représente

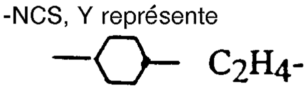

et/ou m = 2 et/ou un des restes $L^1$, $L^2$ et $L^3$ représente le fluor,

ainsi que le diphénylyléthane de formule I36

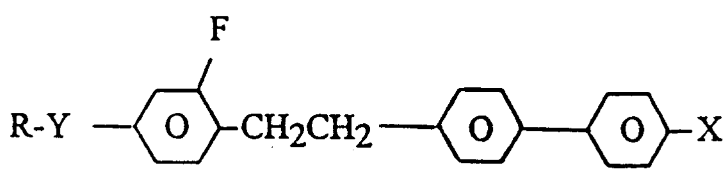 I36

où R et Y ont la signification donnée plus haut et X représente - CN ou -NCS.

**2.** Diphénylyléthanes de formule Ia,

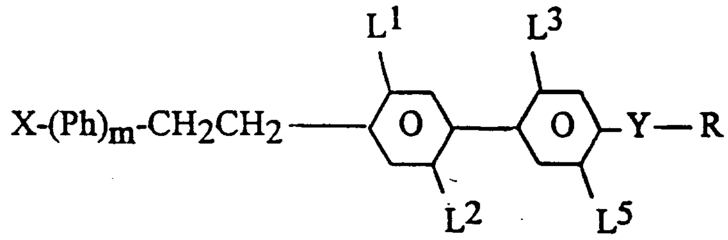

Ia

où

R représente un alkyle, un fluoroalkyle, un alcényle ou un oxa-alkyle,

m est 1 ou 2,

Y représente O, S, CO-O, O-CO, O-CO-O, une liaison simple ou, dans le cas où m = 1, également le trans-1,4-cyclohexylène,

Ph représente chaque fois des restes identiques ou différents choisis dans le groupe constitué par le 1,4-phénylène, le 2-fluoro-1,4-phénylène, le 3-fluoro1,4-phénylène et le 3,5-difluoro-1,4-phénylène,

$L^1$, $L^2$, $L^3$ et $L^5$ représentent chaque fois H ou F,

X représente F, Cl, $-CF_3$, -CN, $-OCF_3$, $-OCHF_2$, -NCS ou - $NO_2$, à la condition que

a) dans le cas où X = -CN, Y représente le trans-1,4-cyclohexylène ou m = 2 et/ou Ph-X représente le 2-fluoro-4-cyanophényle, le 3-fluoro-4-cyanophényle ou le 3,5-difluoro-4-cyanophényle et/ou un ou deux des restes $L^1$, $L^2$, $L^3$ et $L^5$ représentent F, et

b) dans le cas où X = NCS, m = 2 et/ou Ph-X représente le 2-fluoro-4-isothiocyanatophényle, le 3-fluoro-4-isothiocyanatophényle et/ou un ou deux des restes $L^1$, $L^2$, $L^3$ et $L^5$ représentent F et/ou - Y-R représente un alkyle à chaîne droite ayant jusqu'à 3 atomes de C.

**3.** Diphénylyléthanes de formule Ib

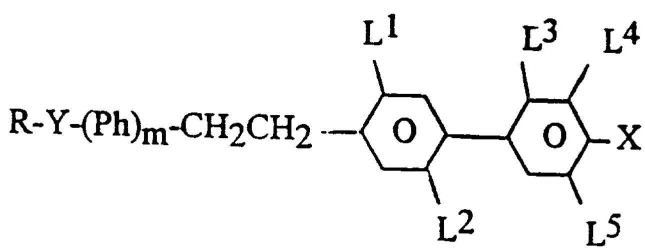

Ib

où

R représente un alkyle, un fluoroalkyle, un alcényle ou un oxa-alkyle,

m est 1 ou 2,

Y représente O, S, CO-O, O-CO, O-CO-O, une liaison simple ou, dans le cas où m = 1, également le trans-1,4-cyclohexylène,

Ph représente chaque fois des restes identiques ou différents choisis dans le groupe constitué par le 1,4-phénylène, le 2-fluoro-1,4-phénylène, le 3-fluoro-1,4-phénylène et le 3,5-difluoro-1,4-phénylène,

$L^1$, $L^3$ et $L^4$ représentent chaque fois H ou un de ces restes est aussi F,

$L^2$ et $L^5$ représentent chaque fois H ou F, et

X représente F, Cl, $-CF_3$, -CN, $-OCF_3$, -NCS ou $-NO_2$, à la condition que

dans le cas où X = F, Cl, -CN ou $-CF_3$, m = 2 et/ou $L^4$ et/ou $L^5$ représentent le fluor (si X = halogène ou $-CF_3$) ou $L^4$ et $L^5$ ou $L^1$ (si X = CN) représentent le fluor.

**4.** Composés selon la revendication 2, caractérisés par la formule partielle Ia1

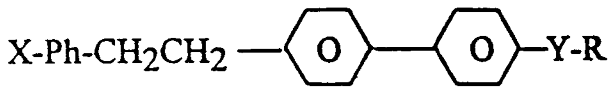

Ia1

où X, Ph et R ont la signification donnée et Y représente O ou une liaison simple.

**5.** Composés selon la revendication 3, caractérisés par la formule partielle Ib1

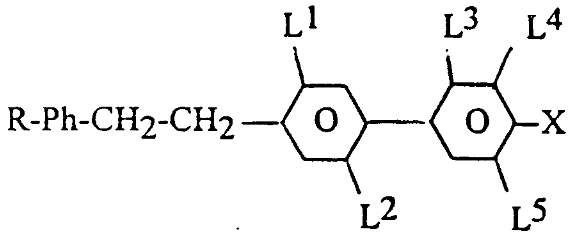

Ib1

où R, Ph, $L^1$, $L^2$, $L^3$, $L^4$, $L^5$ et X ont la signification donnée.

**6.** Composés selon la revendication 1, caractérisés en ce que X représente F, Cl, $-CF_3$, $-OCF_3$ ou $-OCHF_2$.

**7.** Composés de formule Ia4

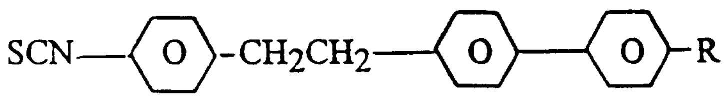

Ia4

où R représente le méthyle, l'éthyle ou le n-propyle.

**8.** Composés selon la revendication 2, caractérisés par la formule

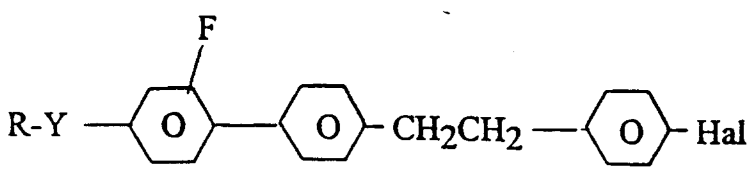

I2

où Hal représente F ou Cl et R et Y ont la signification donnée.

**9.** Composés selon la revendication 2, caractérisés par les formules

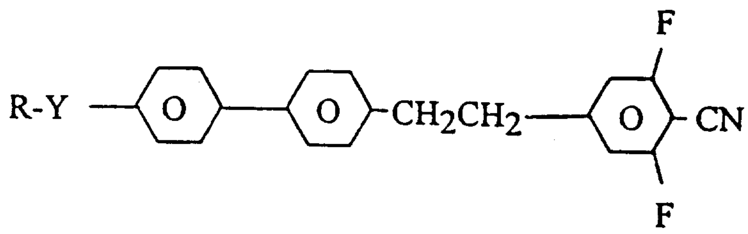

I10a

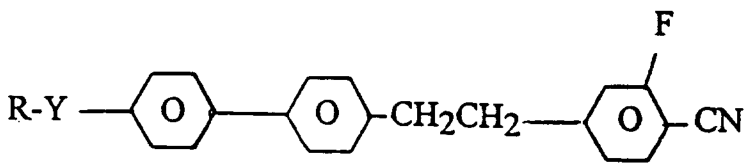

I10b

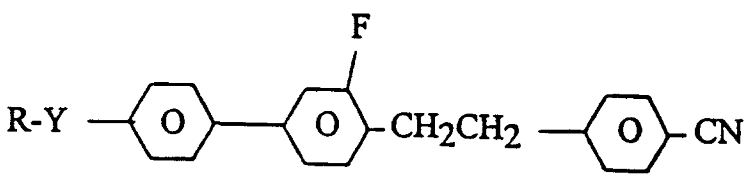

I11a

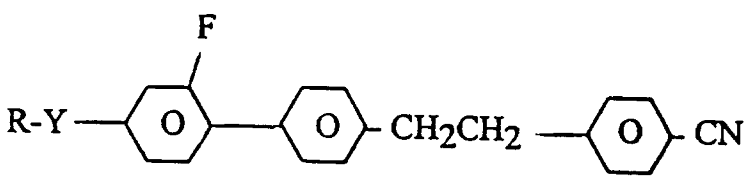

I11b

où R et Y ont la signification donnée.

**10.** Composés selon la revendication 1, caractérisés par les formules

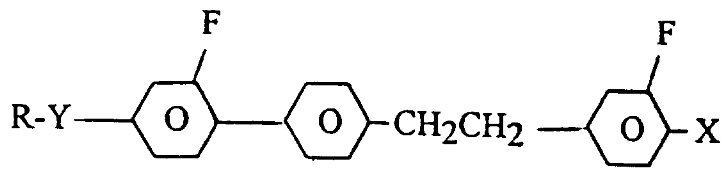

I13

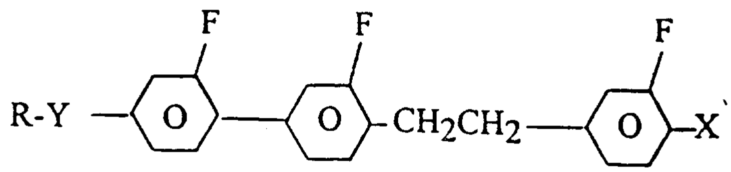

I14

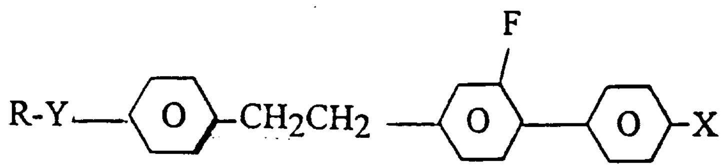

I18

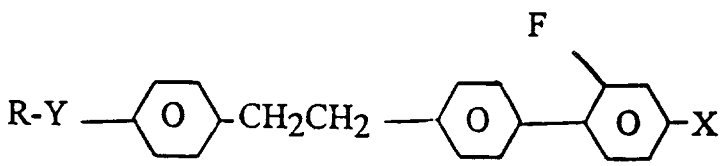

I19

où R, Y et X ont les significations données.

**11.** Composés selon la revendication 3, caractérisés par la formule

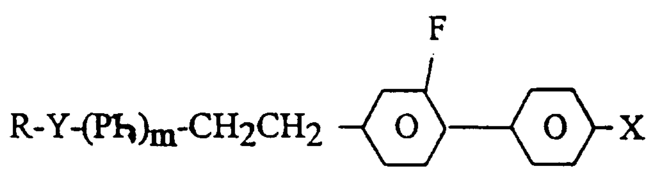

V

où X représente F, Cl, $CF_3$, $OCF^3$ ou $OCHF_2$ et R, Y, Ph et m ont la signification donnée.

**12.** Composés selon la revendication 1, caractérisés en ce que $R^1$-$(Ph)_m$-et $L^1$ à $L^5$ ont les significations suivantes :

| $R^1$-(Ph)$_m$- | $L^1$ | $L^2$ | $L^3$ | $L^4$ | $L^5$ |
|---|---|---|---|---|---|
| R-Y-(Ph)$_m$- | H | F | H | H | H |
| R-Y-(Ph)$_m$- | H | H | H | F | H |
| R-Y-(Ph)$_m$- | H | H | H | F | F |
| R-Y-(Ph)$_m$- | H | F | H | F | H |
| R-Y-(Ph)$_m$- | H | F | H | F | F |
| R-Y 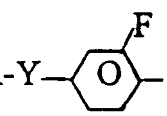 | H | H | H | H | H |
| R-Y 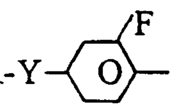 | H | F | H | H | H |
| X-(Ph)$_m$ | H | H | F | H | H |
| X-(Ph)$_m$ | F | H | H | H | H |
| X-(Ph)$_m$ | F | H | F | H | H |
| X 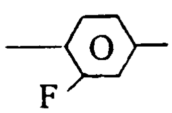 | H | H | H | H | H |
| X 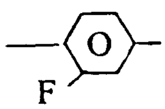 | H | H | F | H | H |

**13.** Utilisation des composés de formule I selon la revendication 1 comme composants à phases de cristaux liquides.

**14.** Phase de cristaux liquides avec au moins deux composants de cristaux liquides, caractérisée en ce qu'elle comporte au moins un composé de formule I.

**15.** Elément d'affichage de cristaux liquides, caractérisé en ce qu'il comporte une phase de cristaux liquides selon la revendication 14.